(19) Europäisches Patentamt
European Patent Office
Office européen des brevets

(11) **EP 2 498 671 B1**

(12) **FASCICULE DE BREVET EUROPEEN**

(45) Date de publication et mention
de la délivrance du brevet:
**20.05.2020 Bulletin 2020/21**

(21) Numéro de dépôt: **10787511.4**

(22) Date de dépôt: **09.11.2010**

(51) Int Cl.:
*A61B 3/103* (2006.01)    *A61B 3/11* (2006.01)
*A61B 3/14* (2006.01)    *A61B 3/113* (2006.01)

(86) Numéro de dépôt international:
**PCT/FR2010/000747**

(87) Numéro de publication internationale:
**WO 2011/058244 (19.05.2011 Gazette 2011/20)**

(54) **PROCÉDÉ ET DISPOSITIF DE MESURE AUTOMATIQUE D'AU MOINS UNE CARACTÉRISTIQUE DE RÉFRACTION DES DEUX YEUX D'UN INDIVIDU**

VERFAHREN UND VORRICHTUNG ZUR AUTOMATISCHEN MESSUNG MINDESTENS EINER BRECHUNGSEIGENSCHAFT BEIDER AUGEN EINER PERSON

METHOD AND DEVICE FOR AUTOMATICALLY MEASURING AT LEAST ONE REFRACTIVE CHARACTERISTIC OF BOTH EYES OF PERSON

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.11.2009 FR 0905467**

(43) Date de publication de la demande:
**19.09.2012 Bulletin 2012/38**

(73) Titulaire: **Essilor International**
**94220 Charenton-le-Pont (FR)**

(72) Inventeurs:
• **BARANTON, Konogan**
**94220 Charenton-Le-Pont (FR)**
• **DROBE, Bjorn**
**94220 Charenton-Le-Pont (FR)**

(74) Mandataire: **Jacobacci Coralis Harle**
**32, rue de l'Arcade**
**75008 Paris (FR)**

(56) Documents cités:
**WO-A2-2009/024681    DE-A1- 19 719 694**
**US-A- 4 834 528    US-A1- 2003 108 350**
**US-A1- 2009 079 937    US-A1- 2010 013 966**
**US-B1- 6 616 277    US-B1- 6 663 242**

EP 2 498 671 B1

**Description**

DOMAINE TECHNIQUE AUQUEL SE RAPPORTE L'INVENTION

La présente invention concerne de manière générale un procédé de mesure de caractéristiques optiques des yeux d'un individu. Il concerne plus particulièrement un procédé de mesure automatique d'au moins une caractéristique de réfraction des deux yeux d'un individu.

L'invention concerne également un dispositif de mesure permettant la mise en oeuvre d'un tel procédé.

ARRIERE-PLAN TECHNOLOGIQUE

Les caractéristiques de réfraction des yeux d'un individu sont des grandeurs qui jouent un rôle essentiel dans la fabrication d'une paire de lunettes de correction visuelle pour cet individu.

Différents procédés et dispositifs sont actuellement connus pour déterminer les caractéristiques de réfraction d'un oeil d'un individu. Ils sont notamment basés sur la technique de la photoréfraction et sur une technique appelée « skiascopie ». On connaît par exemple du document US2009/079937 un procédé et un dispositif pour déterminer l'amétropie des veux d'une personne.

D'autres exemples des dispositifs pour la détermination de la réfraction oculaire sont présentés dans les documents US6616277 B1 ou WO2009/024681 A. Cependant, ces méthodes et les dispositifs associés sont fastidieux à mettre en oeuvre.

Les dispositifs manuels avec lesquels l'opérateur observe à l'oeil nu ledit reflet rétinien sont peu précis. La précision est améliorée par l'utilisation d'un détecteur, mais les mesures restent longues à réaliser, car chaque oeil de l'individu doit faire l'objet d'une mesure distincte : seul le reflet rétinien d'un seul des deux yeux de l'individu est observé à la fois.

En outre, les dispositifs connus comportent des moyens de contrainte pour imposer une posture prédéterminée de la tête et/ou des yeux de l'individu dans un référentiel lié au dispositif de capture d'image, par exemple par un appui frontal et une mentonnière, et/ou des moyens de contrainte de la direction du regard, par exemple par la présence d'une cible à fixer du regard.

Les mesures des caractéristiques de réfraction réalisées grâce aux méthodes et dispositifs existants ne sont donc pas réalisées dans des conditions de posture naturelle et de direction du regard naturelle et ne permettent pas de prendre en compte la vision binoculaire de l'individu.

Ces mesures ne permettent pas, par exemple, de déterminer les caractéristiques de réfraction de l'oeil de l'individu en conditions de vision de près ou intermédiaire.

Or les caractéristiques de réfraction de l'oeil peuvent être sensiblement modifiées selon les conditions de vision, la tâche à effectuer par l'individu, la posture et la direction de regard de l'individu.

OBJET DE L'INVENTION

Un but de la présente invention est de fournir un procédé et un dispositif permettant de réaliser une mesure des caractéristiques de réfraction des deux yeux d'un individu de manière rapide et fiable, dans une posture naturelle et confortable non contrainte de l'individu et dans des conditions visuelles déterminées. Cette posture naturelle correspond à la posture la plus ergonomique et la moins fatigante pour l'individu. En d'autres termes, elle correspond à la posture assumée habituellement par l'individu.

A cet effet, on propose selon l'invention un procédé de mesure automatique d'au moins une caractéristique de réfraction des deux yeux d'un individu, conforme à la revendication 1.

L'invention concerne également un procédé de mesure automatique d'au moins une caractéristique de réfraction des deux yeux d'un individu dans des conditions visuelles prédéfinies, conforme à la revendication 2.

Ainsi, la mesure de la caractéristique de réfraction des yeux de l'individu, par exemple le signe de l'amétropie ou la valeur de cette amétropie, c'est-à-dire leurs puissances sphériques et/ou cylindriques, est réalisée dans une posture naturelle et confortable de l'individu, ce qui permet de déterminer cette caractéristique de réfraction dans des conditions réelles de vision de l'individu. La précision de ces mesures est ainsi améliorée. En outre, cette caractéristique de réfraction est mise en relation avec un paramètre de direction du regard lié à la direction du regard de l'individu.

Ce paramètre de direction du regard est un paramètre de posture de la tête de l'individu qui permet par exemple de déterminer précisément la position relative de la tête du porteur par rapport à l'appareil de capture d'image, quelle que soit la manière dont l'individu tient cet appareil.

On entend ici par « lié à la direction du regard » qu'il existe une relation mathématique entre ledit paramètre de direction du regard et ladite direction du regard de l'individu permettant de calculer l'un à partir de l'autre.

Le lien peut être direct, le paramètre de direction du regard consistant alors typiquement en un suivi par reconnaissance d'images de l'orientation des yeux, ou indirect, le paramètre mesuré concernant alors exclusivement la tête et consistant en une ou plusieurs coordonnées de position et/ou d'orientation de la tête. Cela sera décrit ultérieurement plus en détails dans un mode de réalisation particulier de l'invention.

Ce procédé permet ainsi la réalisation d'une cartographie associant, à chaque valeur de la caractéristique de réfraction des yeux déterminée, la direction du regard de l'individu correspondante. Cela permet notamment de distinguer les valeurs de la caractéristique de réfraction qui sont associées à une vision de près ou intermédiaire et celles associées à une vision de loin.

On entend ici par vision de près, des conditions dans lesquelles l'individu fixe regarde un objet distant de 25 à 69 centimètres, par vision intermédiaire, des conditions dans lesquelles l'individu fixe regarde un objet distant de 70 à 199 centimètres, et par vision de loin des conditions dans lesquelles l'individu fixe regarde un objet distant d'une distance supérieure ou égale à 200 centimètres.

L'individu ajuste lui-même, manuellement ou par commande à distance, la position et l'orientation de l'appareil de capture d'image, ou donne des instructions pour que cet ajustement soit réalisé selon son souhait.

Alternativement, si la position de l'appareil de capture d'image est fixé, l'individu se place dans une posture de son souhait face à cet appareil.

Selon ce procédé, les caractéristiques de réfraction des deux yeux de l'individu sont mesurées simultanément. La mesure est donc simple et rapide à mettre en oeuvre. Elle permet en outre de prendre en compte la réalité du système d'accommodation et de convergence des yeux en vision binoculaire.

Selon une première caractéristique avantageuse de l'invention, il est en outre prévu l'étape suivante :
e) déterminer, à partir de ladite au moins une image capturée, une largeur ou une répartition de luminance de l'image du reflet rétinien de la source lumineuse et déterminer ladite caractéristique de réfraction des yeux de l'individu en fonction de cette largeur ou de cette répartition de luminance.

Alors avantageusement, les deux yeux étant éclairés simultanément, les images des reflets rétiniens de la source lumineuse sur la rétine des deux yeux sont capturées simultanément et, à l'étape e), la largeur ou la répartition de luminance de l'image du reflet rétinien de la source lumineuse est déterminée au moins à partir desdits signaux relatifs aux reflets rétiniens des deux yeux capturés simultanément dans chaque posture de la tête de l'individu par rapport à ladite source lumineuse.

Ce mode de réalisation de l'invention permet avantageusement de déterminer ladite caractéristique de réfraction par une méthode de photoréfraction.

Selon une autre caractéristique avantageuse de l'invention, les deux yeux étant éclairés simultanément, les images des reflets rétiniens de la source lumineuse sur la rétine des deux yeux sont capturées simultanément et à l'étape e), la largeur ou la répartition de luminance de l'image du reflet rétinien de la source lumineuse est déterminée au moins à partir desdits signaux relatifs aux reflets rétiniens des deux yeux capturés simultanément dans chaque posture de la tête de l'individu par rapport à ladite source lumineuse.

Alors, on déduit une valeur de l'amétropie des yeux de l'individu de la largeur ou de la répartition de luminance de l'image du reflet rétinien de la source lumineuse.

On peut ainsi déterminer une valeur précise de l'amétropie des deux yeux.

Selon une autre caractéristique avantageuse de l'invention, au moins deux images du reflet rétinien de ladite source lumineuse sur la rétine des deux yeux de l'individu étant capturées, le procédé comporte en outre l'étape suivante :
e') déterminer, à partir desdites au moins deux images capturées, un déplacement de l'image du reflet rétinien de la source lumineuse (20),
ladite caractéristique de réfraction des yeux de l'individu étant déterminée en fonction de ce déplacement.

Selon une autre caractéristique avantageuse de l'invention,
les postures relatives de la tête de l'individu par rapport à ladite source lumineuse étant différentes lors des deux captures d'image,
à l'étape c), on mesure, lors desdites captures d'image, au moins un paramètre de posture de la tête de l'individu dans le référentiel de l'appareil de capture d'image,
à l'étape e'), on compare le déplacement de l'image du reflet rétinien déterminé à la différence des postures relatives de la tête de l'individu par rapport à la source lumineuse entre les deux captures d'image, en fonction dudit paramètre de posture de la tête mesuré à l'étape c),
ladite caractéristique de réfraction des yeux de l'individu étant déduite de cette comparaison.

Ce mode de réalisation de l'invention permet avantageusement de déterminer ladite caractéristique de réfraction par une méthode de skiascopie.

Avantageusement alors, le signe de l'amétropie des yeux de l'individu est déduit de ladite comparaison.

Selon une autre caractéristique avantageuse de l'invention, les deux yeux étant éclairés simultanément, les images des reflets rétiniens de la source lumineuse sur la rétine des deux yeux sont capturées simultanément et selon lequel, à l'étape e'), le déplacement de l'image du reflet rétinien de la source lumineuse est déterminé au moins à partir desdits signaux relatifs aux reflets rétiniens des deux yeux capturés simultanément dans chaque posture de la tête de l'individu par rapport à ladite source lumineuse.

De préférence, lors de la capture d'image, un support sur lequel sont montés ladite source lumineuse et ledit appareil de capture d'image est porté à la main par ledit individu. Ceci permet d'assurer que l'individu se trouve dans une position naturelle et confortable pour lui.

Selon une autre caractéristique avantageuse et non limitative de l'invention, il est prévu une étape de vérification antérieure ou postérieure à l'étape de capture d'image, de ce que ladite au moins une image est capturée lorsque l'individu regarde au voisinage, à l'intérieur d'une plage déterminée, d'une pupille de l'appareil de capture d'image.

Selon une autre caractéristique avantageuse et non limitative de l'invention, ledit paramètre de direction du regard consiste exclusivement en un paramètre de posture de la tête de l'individu mesuré lorsque l'individu fixe du regard la pupille de l'appareil de capture d'image.

Selon une autre caractéristique avantageuse et non limitative de l'invention, lors d'une étape préliminaire à la capture d'au moins une image, on place sur le visage dudit individu un équipement de correction visuelle et selon lequel on corrige les signaux représentatifs de ladite image capturée et/ou la caractéristique de réfraction déduite de ces signaux en fonction de la puissance et de la déviation de l'équipement de correction visuelle.

Les images capturées lors de capture d'image du procédé selon l'invention peuvent alors être traitées pour déterminer d'autres paramètres physico-morphologiques de l'individu, comme la hauteur entre le bord inférieur du cercle d'une monture de lunettes et le centre de la pupille des yeux de l'individu.

Selon une autre caractéristique avantageuse et non limitative de l'invention, à l'étape c), on calcule la direction du regard de l'individu à partir des signaux représentatifs des images des reflets cornéens des deux yeux.

En outre, selon une variante le procédé selon l'invention comporte les étapes suivantes:

> r) avant ou pendant la capture d'au moins une image, afficher devant les yeux de l'individu une image présentant un flou correspondant à un filtrage passe-bas par un filtre de fréquence de coupure donnée des fréquences spatiales de cette image,
> t) mettre en mémoire la caractéristique de réfraction déterminée en relation avec la valeur de la fréquence de coupure du filtre passe-bas utilisé à l'étape r),
> u) réitérer au moins les étapes b), c), d), f), r) et t) ou les étapes b), c), i), j), r) et t) pour différentes fréquences de coupure du filtre de l'image.

Ce procédé permet ainsi de réaliser, pour différentes valeurs de la fréquence de coupure du filtre utilisé à l'étape r), donc pour différents degrés de flou de l'image, la mesure d'une caractéristique de réfraction des yeux de l'individu lorsqu'il est dans les conditions de visualisation de l'image floue en question.

Il est alors avantageusement possible de déterminer l'évolution de la caractéristique de réfraction des yeux de l'individu lorsque le degré de flou de l'image varie.

Il est en outre possible de caractériser la perception du flou par l'individu pour différents degrés de flou de l'image et de déterminer l'évolution de la perception du flou lorsque le degré de flou de l'image varie.

Cette variante permet ainsi la réalisation d'un test psychophysique.

Selon une autre variante du procédé :

- lors de ladite étape de capture d'image, on capture successivement au moins deux images comportant chacune les images des deux reflets rétiniens de ladite source lumineuse sur les rétines des deux yeux de l'individu,
- on détermine, à partir de l'une de ces deux images, ladite caractéristique de réfraction de l'un des deux yeux de l'individu et à partir de l'autre image, la caractéristique de réfraction de l'autre oeil de l'individu.

L'invention concerne également un dispositif de mesure automatique d'au moins une caractéristique de réfraction des deux yeux d'un individu, conforme à la revendication 16.

Selon une caractéristique avantageuse et non limitative de l'invention, lesdits moyens de mesure dudit paramètre de posture de la tête de l'individu comportent un élément de repérage de la position de la tête de l'individu adapté à être monté sur la tête ou sur les lunettes de l'individu ou un dispositif de mesure à ultra-son, comprenant au moins un émetteur et un récepteur d'ultra-son ou un dispositif de mesure à capteurs électromagnétiques.

Ce dispositif de mesure autorise une mesure à distance rapide et précise du paramètre de posture de la tête de l'individu.

Selon une autre caractéristique avantageuse et non limitative de l'invention, la source lumineuse comporte une pluralité de sources lumineuses secondaires dont les positions relatives les unes par rapport aux autres sont connues et qui peuvent chacune être allumées indépendamment des autres.

L'activation d'une source lumineuse différente à chaque capture d'image assure que, si la tête de l'individu ne bouge pas, que les postures relatives de la tête de l'individu par rapport à ladite source lumineuse soient différentes lors des captures d'image.

Alternativement, la source lumineuse est mobile par rapport au dispositif de capture d'image.

DESCRIPTION DETAILLEE D'UN EXEMPLE DE REALISATION

La description qui va suivre, en regard des dessins annexés, donnée à titre d'exemple non limitatif, fera bien comprendre en quoi consiste l'invention et comment elle peut être réalisée.

Sur les dessins annexés :

- la figure 1 est une vue en perspective d'un mode de réalisation du mesure ;
- la figure 2 est une vue schématique en perspective des yeux de l'individu de la figure 1 lorsque l'individu regarde dans la direction du dispositif de mesure de la figure 1 lors d'une première capture d'image;
- la figure 3 est une vue schématique dans le plan de capture d'image de la première image capturée en référence à la figure 2;
- la figure 4 est une vue schématique en perspective des yeux de l'individu de la figure 1 lorsque l'individu regarde dans la direction du dispositif de mesure de la figure 1 lors d'une deuxième capture d'image;
- la figure 5 est une vue schématique dans le plan de capture d'image de la deuxième image capturée en référence à la figure 4;

- la figure 6 est une vue de profil de l'individu portant à la main le dispositif de mesure de la figure 1;
- la figure 7 est une vue schématique de dessus, dans un plan horizontal des yeux de l'individu et du dispositif de mesure de la figure 1.

Il s'agit, globalement, de réaliser la mesure automatique d'au moins une caractéristique de réfraction des deux yeux d'un individu dans une posture naturelle, dans des conditions visuelles connues ou déterminées. Ainsi, on réalise une mesure d'un paramètre de direction du regard correspondant à chaque capture d'image, de manière, soit à enregistrer ce paramètre de direction du regard en relation avec la caractéristique de réfraction des yeux correspondante, soit à déclencher les captures d'image lorsque le paramètre de direction du regard mesuré indique que l'individu se trouve dans des conditions visuelles prédéterminées d'intérêt, soit à sélectionner les images capturées dans des conditions visuelles d'intérêt après la capture d'une série d'images pour déterminer la caractéristique de réfraction des yeux dans ces conditions.

On s'intéresse notamment aux conditions visuelles dans lesquelles l'individu est en vision de près ou intermédiaire. En effet, les caractéristiques de réfraction de l'oeil peuvent être sensiblement modifié par le phénomène d'accommodation de l'oeil qui accompagne la vision de près ou intermédiaire.

Dans l'exemple décrit ci-après, cette mesure repose sur le principe de la skiascopie, décrite par exemple dans le document intitulé « Principles of retinoscopy » publié par M. Klein en 1944 dans la revue The British Journal of Ophtalmology.

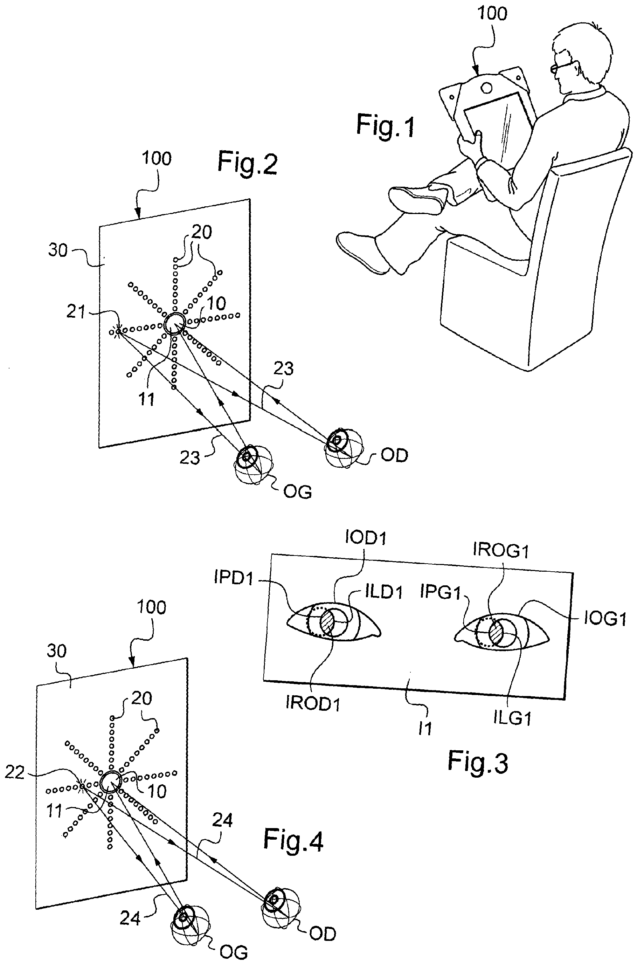

Un exemple de dispositif de mesure 100 selon l'invention est représenté sur les figures 1, 2 et 4. Ce dispositif de mesure 100 comporte ici un boîtier 30 qui sert de support à un dispositif de capture d'image 10 et à des moyens d'éclairage 20 de la tête de l'individu.

Le boîtier 30 présente avantageusement la forme d'une tablette rectangulaire.

Il s'agit de déterminer par exemple l'amétropie objective des yeux de l'individu en déterminant au moins l'une des caractéristiques de réfraction suivantes: signe de l'amétropie, puissance sphérique, puissance cylindrique et position de l'axe du cylindre.

Le signe de l'amétropie est négatif en cas de myopie et positif en cas d'hypermétropie. La valeur de l'amétropie correspond aux puissances sphériques et cylindriques de l'oeil. Elle est déterminée suivant différents méridiens de l'oeil. La puissance cylindrique et l'axe du cylindre caractérisent l'astigmatisme de l'oeil.

Ces caractéristiques de réfraction sont essentielles dans le choix de l'équipement optique adapté à l'individu, puisqu'elles permettent de déterminer le type de lentille ophtalmique adaptée à la correction de l'amétropie de l'individu : convergente, divergente ou torique, et la puissance de cette lentille ophtalmique.

L'individu est dans une posture naturelle, débout ou assis, comme représenté sur la figure 1. Sa posture par rapport au dispositif de mesure 100 n'est pas contrainte par une mentonnière ou un appui frontal. On entend par posture naturelle la posture de l'individu qui est la plus confortable, la plus ergonomique et la moins fatigante pour lui.

Dans une étape a), au moins une posture de la tête de l'individu est ajustée dans un référentiel lié à l'appareil de capture d'image, selon le souhait dudit individu.

Cela peut être réalisé simplement manuellement par l'individu lui-même, qui se place librement et dispose selon son désir le dispositif de capture d'image 100, notamment dans un premier mode de réalisation où l'individu porte le dispositif de mesure à la main, comme représenté par exemple sur la figure 1.

Dans un deuxième mode de réalisation dans lequel le dispositif de capture d'image est posé sur un support ou monté sur un pied, l'individu peut réaliser cet ajustement à distance à l'aide d'une télécommande permettant de modifier le positionnement du dispositif de mesure, ou par des instructions vocales transmise à un opérateur qui modifie la position du dispositif de mesure selon ces instructions.

Enfin, selon un troisième mode de réalisation, le dispositif de mesure est fixe, et l'individu se place comme il le souhaite par rapport à ce dispositif de mesure 100.

Quel que soit le mode de réalisation, il est ainsi possible de réaliser les captures d'image de la tête de l'individu dans une position naturelle non contrainte. Cela permet de déterminer les caractéristiques de réfraction des yeux de l'individu dans des conditions réelles de vision.

En variante, une tâche visuelle particulière peut être attribuée à l'individu, par exemple, la lecture d'un texte placé à proximité de la pupille de l'appareil de capture d'image. Cela permet alors de réaliser les captures d'images, et donc de déterminer les caractéristiques de réfraction des yeux de l'individu dans la posture qu'il adopte naturellement pour réaliser cette tâche visuelle.

Dans une étape b), l'opérateur éclaire les deux yeux de l'individu au moyen d'au moins une source lumineuse, dont la position, dans un référentiel (O, X, Y, Z) lié à l'appareil de capture d'image, est connue.

Le référentiel (O,X,Y,Z) lié à l'appareil de capture d'image 10 est par exemple ici centré sur la pupille de cet appareil de capture d'image. Les axe (O,X), (O,Y) s'étendent dans le plan de capture d'image PCI, qui est le plan du capteur de l'appareil de capture d'image, ici représenté par le plan moyen du support 30. L'axe (OZ) est l'axe optique de l'appareil de capture d'image, qui est perpendiculaire au plan de capture d'image PCI.

Dans l'exemple représenté sur les figures 1, 2 et 4, les moyens d'éclairage 20 du dispositif de mesure 100 comportent une pluralité de sources lumineuses 20. Ces sources lumineuses sont de préférence de très petite taille, par exemple quasi-ponctuelles. Elles présentent par exemple chacune une largeur d'environ 1 mm de diamètre. Il s'agit ici de diodes 20 électroluminescentes. Chacune de ces diodes peut être allumée indépendamment des autres et éclaire les deux yeux de l'individu. Ces diodes constituent des sources lumineuses divergentes. Elle éclairent ainsi avantageusement un angle solide très étendu, de sorte que la tête de l'individu est largement éclairée.

Les sources lumineuses 20 envisagées ici sont des sources primaires, cependant, en variante, il peut s'agir de sources secondaires obtenues par réflexion ou déviation des rayons lumineux issus de telles sources primaires.

Ici, les diodes sont disposées en face avant du boîtier 30 servant également de support à l'appareil de capture d'image 10, et la position de chacune de ces diodes dans le référentiel de l'appareil de capture d'image est donc connue par construction et fixe.

Les diodes sont ici disposées dans le plan de la face avant du boîtier 30, alignées selon quatre directions d'alignement qui se croisent en un point où est disposée la pupille 11 de l'appareil de capture d'image 10.

Alternativement, on peut envisager d'utiliser tout autre type de source lumineuse connue de l'homme du métier. Il est en particulier possible d'utiliser une source lumineuse unique pouvant être déplacée par rapport à la tête de l'individu, dans le référentiel de l'appareil de capture d'image. Le dispositif de mesure comporte alors des moyens de détermination de la position de la source lumineuse dans ce référentiel.

Dans une étape de capture d'image, l'appareil de capture d'image 10 capture au moins une image et de préférence deux images du reflet rétinien de ladite source lumineuse sur la rétine des deux yeux de l'individu, chaque image étant capturée dans un plan de capture d'image correspondant, et les postures relatives de la tête de l'individu par rapport à ladite source lumineuse étant différentes lors des deux captures d'image.

La capture des images par le dispositif de capture d'image est déclenchée par l'opérateur ou automatiquement par exemple par un dispositif électronique et informatique.

Le dispositif électronique et informatique comporte par exemple un ordinateur sur lequel est installé un logiciel d'acquisition d'image et de traitement de l'image acquise.

Le dispositif de mesure 100 possède de préférence des moyens pour sa communication avec cet ordinateur équipé de moyens de communication correspondants. Les moyens de communication du dispositif de mesure 100 et de l'ordinateur sont du type filaires ou sans fil et, étant d'une conception courante quelconque, ne seront pas décrits. Avantageusement, ces moyens de communication s'intègrent à une architecture de réseau commune ou de liaison point à point permettant à l'appareil de capture d'image de communiquer avec plusieurs ordinateurs.

Le dispositif électronique et informatique peut ainsi déclencher à des intervalles prédéterminés par l'opérateur, les captures d'image. Le dispositif de mesure 100 transmet les images capturées à ce dispositif électronique et informatique, qui reçoit également les mesures du paramètre de posture de la tête de l'individu réalisées à l'étape c) décrite plus loin.

En variante, on peut prévoir que le dispositif électronique et informatique soit un système autonome qui comporte, d'une part, un écran d'affichage pour communiquer les images capturées et/ou les résultats obtenus aux étapes de traitement de ces images et, d'autre part, une connectique pour permettre de communiquer ces résultats à d'autres appareils. On peut également prévoir dans le cas d'un système autonome de traitement que ce système soit intégré ou non au dispositif de mesure 100.

Ces deux captures d'image ont par exemple lieu dans les configurations représentées sur les figures 2 et 4. Sur ces figures, on a représenté schématiquement les yeux de l'individu pendant la capture de ces deux images, par rapport au dispositif de mesure 100.

Pendant une première capture d'image, représentée sur la figure 2, une première 21 des diodes 20 du dispositif de mesure 100 est allumée et éclaire simultanément l'oeil droit OD et l'oeil gauche OG de l'individu, comme représenté par les rayons lumineux 23. Ces rayons lumineux 23 éclairent la rétine de chaque oeil OD, OG de l'individu, qui se comporte alors comme une source lumineuse secondaire : les rayons lumineux sont réfléchis de façon diffuse par la rétine et retraversent l'oeil. Une partie des rayons lumineux 23 réfléchis de façon diffuse par la rétine sort de l'oeil OD, OG correspondant et est reçue par la pupille 11 de l'appareil de capture d'image 10.

De même, pendant une deuxième capture d'image, représentée sur la figure 4, une deuxième diode 22 du dispositif de mesure 100 est allumée et éclaire simultanément l'oeil droit OD et l'oeil gauche OG de l'individu, comme représenté par les rayons lumineux 24. Ces rayons lumineux 24 éclairent la rétine de chaque oeil OD, OG de l'individu. Une partie des rayons lumineux 24 réfléchis par la rétine sort de l'oeil OD, OG correspondant et est reçue par la pupille 11 de l'appareil de capture d'image 10.

Les images I1 et I2 correspondantes, capturées par l'appareil de capture d'image 10, comportent ainsi l'image du reflet IROD1, IROG1, IROD2, IROG2 de la première et de la deuxième diode 21, 22 correspondante sur la rétine de chaque oeil OD, OG de l'individu.

En général, seule une partie de cette image du reflet IROD1, IROG1, IROD2, IROG2 rétinien est visible à travers la pupille 11 de chaque oeil OD, OG. La partie de cette image qui est visible est celle comprise dans l'espace tronconique compris entre la pupille de l'individu et la pupille de l'appareil de capture d'image.

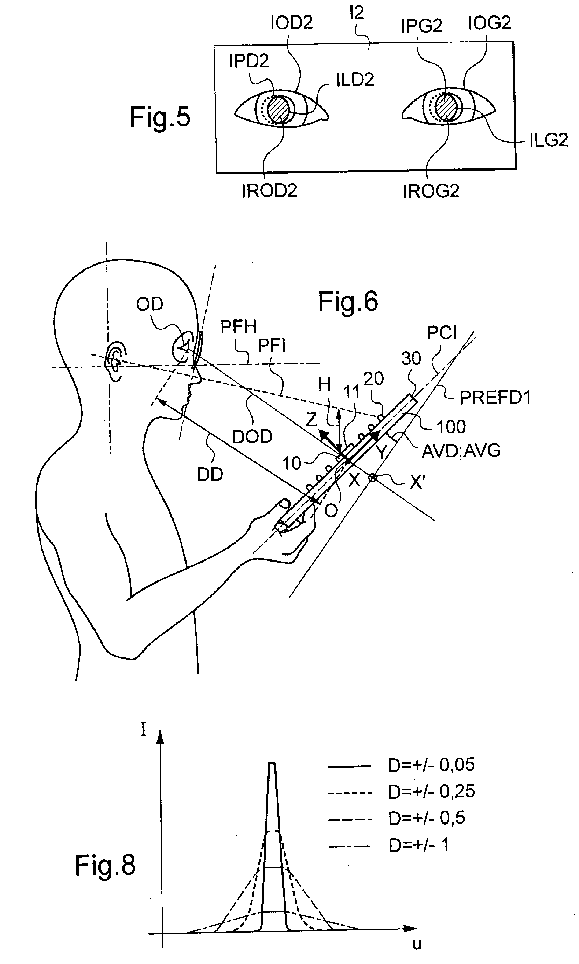

Ici, l'image du reflet IROD1, IROG1, IROD2, IROG2 rétinien est représenté hachurée sur l'image de l'oeil IOD1, IOG1, IOD2, IOG2 correspondant (figures 3 et 5). Le contour de cette image du reflet qui n'est pas visible sur l'image capturée est représenté en traits pointillés.

Le dispositif électronique et informatique commande en pratique la capture d'une série d'images parmi lesquelles ne sont retenues que celles sur lesquelles l'image du reflet rétinien est visible. En particulier, le dispositif électronique et informatique sélectionne deux images correspondant à l'entrée et à la sortie de l'image de cet espace tronconique, ce couple d'image donnant accès à un maximum d'information.

Dans cet exemple, la posture de la tête de l'individu dans le référentiel de l'appareil de capture d'image reste la même lors des captures d'image, mais les postures relatives de la tête de l'individu par rapport à ladite source lumineuse sont différentes lors des deux captures d'image puisqu'une diode différente est allumée lors de chaque capture d'image. On peut par exemple pour cela capturer les deux images à un très faible intervalle de temps, de manière à ce que tout mouvement de l'individu soit négligeable dans cet intervalle de temps. Cet intervalle de temps est par exemple compris entre 2 et 100 millisecondes.

La comparaison des images I1 et I2 représentées schématiquement sur les figures 3 et 5 montrent que la position de l'image du reflet IROD1, IROG1 de la diode 21 sur l'image I1 est différente de la position de l'image du reflet IROD2, IROG2 de la diode 22 sur l'image I2. En d'autres termes, lorsque la position de la source lumineuse allumée lors de la capture d'image varie, la position de l'image du reflet de cette source varie.

Ici, la position de l'image du reflet IROD1, IROG1, IROD2, IROG2 de chaque diode imagée à travers la pupille de l'oeil correspondant est matérialisée par la limite ILD1, ILG1, ILD2, ILG2 entre la zone éclairée de l'image de la pupille IPD1, IPG1, IPD2, IPG2 de l'individu, c'est-à-dire le reflet rétinien de la source lumineuse allumée, représenté par des hachures sur les figures 3 et 5, et la zone sombre de la pupille qui n'est pas éclairée par la diode 21, 22. Cette limite ILD1, ILG1, ILD2, ILG2 correspond au bord de l'image du reflet IROD1, IROG1, IROD2, IROG2 de la source lumineuse.

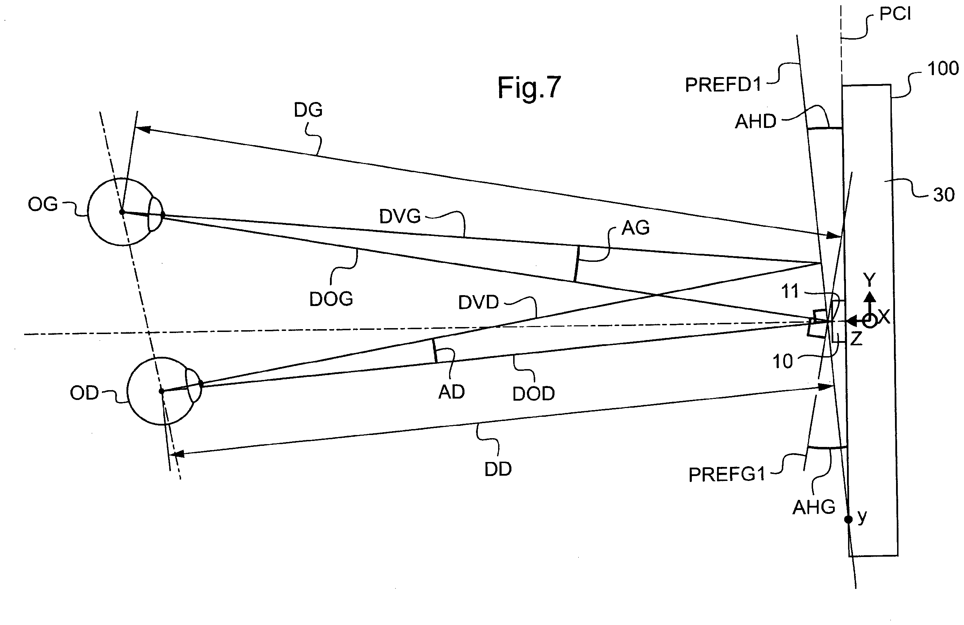

L'étape de capture d'image est réalisée lorsque l'individu regarde au voisinage de la pupille 11 de l'appareil de capture d'image 10. Des droites de visée DVD, DVG suivant la direction du regard de chaque oeil de l'individu forment alors de préférence un angle AD, AG inférieur ou égal à une valeur seuil inférieure à 10 degrés avec une droite d'observation DOD, DOG reliant l'oeil OD, OG à la pupille 11 de l'appareil de capture d'image 10 (figure 7). Cette condition permet d'assurer que la pupille de l'oeil de l'individu est imagée de façon satisfaisante, de manière à ce que les reflets rétiniens de la source sur les deux yeux de l'individu soient visibles sur les deux images capturées.

En pratique une série de plus de deux images est capturée par l'appareil de capture d'image 10. On sélectionne ensuite les images pour lesquelles l'individu regarde au voisinage, à l'intérieur d'une plage déterminée, de la pupille 11 de l'appareil de capture d'image 10, en calculant la direction du regard de l'individu à partir de la position relative du reflet cornéen de la source lumineuse et de la pupille de l'oeil correspondant.

Cette plage déterminée correspond ici par exemple à la surface du support située autour de la pupille de l'appareil de capture d'image, pour laquelle l'angle AD, AG entre la droite de visée DVD, DVG suivant la direction du regard et la droite d'observation DOD, DOG est inférieur à ladite valeur seuil.

Si plusieurs images sont sélectionnées, le dispositif électronique et informatique peut éventuellement être programmé pour moyenner ces images.

Alternativement, il est également possible de prévoir que le procédé selon l'invention comporte une étape de vérification antérieure à l'étape de capture d'image, de ce que ladite au moins une image est capturée lorsque l'individu regarde au voisinage, à l'intérieur d'une plage déterminée, d'une pupille 11 de l'appareil de capture d'image 10. Seules les images pour lesquelles la direction du regard de l'individu est comprise dans cette plage sont capturées.

Enfin, il est également possible de capturer plusieurs images de la tête de l'individu lors de l'allumage de chacune des deux diodes, ce qui permet par exemple de moyenner les images capturées lors de l'allumage de chaque diode, et/ou de réaliser la capture d'une série d'images correspondant à l'allumage successif de différentes diodes. La précision des résultats obtenus par le traitement des images est alors améliorée.

Dans une étape c), effectuée lors desdites captures d'image, au moins un paramètre de direction du regard de l'individu dans un référentiel lié à sa tête est mesuré.

Ce paramètre de direction du regard consiste ici exclusivement en un paramètre de posture de la tête de l'individu mesuré lorsque l'individu fixe du regard la pupille de l'appareil de capture d'image.

Il s'agit ici d'un paramètre de posture de la tête représentatif de l'abaissement du regard vers la pupille de l'appareil de capture d'image. Le point de visée des yeux étant proche de la pupille de l'appareil de capture d'image, ce paramètre de posture de la tête est lié à la direction du regard, qui peut en être directement déduite par calcul.

Cette mesure est réalisée au moins pour l'une des images de chaque série de deux ou plus images capturées. Elle sera exposée plus en détail ultérieurement.

Elle est réalisée soit simultanément à la capture de l'image, soit dans un intervalle de temps inférieur ou égal à 100 millisecondes avant ou après la capture d'image.

Dans une étape e'), un déplacement de l'image du reflet rétinien de la source lumineuse entre les deux images capturées est déterminé à partir desdits signaux représentatifs desdites images par le dispositif électronique et informatique.

Le dispositif électronique et informatique compare le déplacement de l'image du reflet rétinien déterminé à la différence des postures relatives de la tête de l'individu par rapport à la source lumineuse entre les deux captures d'image et en déduit la caractéristique de réfraction des yeux de l'individu.

Selon une première variante du procédé selon l'invention, dans une étape h), les résultats des mesures du paramètre de direction du regard de l'individu sont mis en mémoire dans le dispositif électronique et informatique en relation avec la caractéristique de réfraction déduite des images capturées dans les conditions visuelles correspondant à cette direction du regard.

Le dispositif électronique et informatique reconstitue alors une cartographie associant la direction du regard de l'individu et la caractéristique de réfraction de l'oeil.

Selon une deuxième variante du procédé selon l'invention, lors de l'étape de capture d'image, le dispositif électronique et informatique ne déclenche la capture des images que si la valeur du paramètre de direction du regard H est située dans un intervalle correspondant auxdites conditions visuelles prédéfinies, par exemple à une vision de près. Le dispositif électronique et informatique détermine alors uniquement les caractéristiques de réfraction des yeux de l'individu lorsque celui-ci se trouve dans lesdites conditions visuelles prédéfinies.

Cette variante n'est évidemment à envisager que dans le cas où le paramètre de direction du regard est obtenu indépendamment de la capture d'une image. Ce sera par exemple le cas lorsque le paramètre de direction du regard peut être directement déduit de la posture de la tête de l'individu, et que la posture de la tête de l'individu est déterminée par un moyen de détermination ne nécessitant pas de capture d'image. On peut également envisager qu'une caméra filme en continue la tête de l'individu et que le calcul du paramètre de direction du regard soit réalisé en parallèle à partir des images filmées, pour déclencher la capture des images aux instants adéquats.

Selon une troisième variante du procédé selon l'invention, le dispositif électronique et informatique sélectionne parmi les images capturées celles pour lesquelles le paramètre de direction du regard mesuré présente une valeur située dans un intervalle correspondant auxdites conditions visuelles prédéfinies, par exemple à une vision de près. Le dispositif électronique et informatique détermine alors les caractéristiques de réfraction des yeux de l'individu à partir de ces images sélectionnées.

En pratique, de préférence, les deux yeux OD ,OG étant éclairés simultanément, les images des reflets rétiniens de la source lumineuse sur la rétine des deux yeux sont capturées simultanément et à l'étape e'), la comparaison est réalisée au moins à partir desdits signaux relatifs aux reflets rétiniens des deux yeux capturés simultanément dans chaque posture de la tête de l'individu par rapport à ladite source lumineuse.

Plus particulièrement, à l'étape c), on mesure par exemple comme représenté sur la figure 6, une distance verticale H entre la pupille 11 de l'appareil de capture d'image 10 et un plan anatomique incliné PFI dans les conditions visuelles de la capture d'image, par exemple la vision de près ou intermédiaire qui correspond à un plan anatomique horizontal PFH dans les conditions de vision de loin.

En variante, on mesure à l'étape c) une distance entre la pupille de l'appareil de capture d'image et un plan anatomique PFH d'horizon sensiblement horizontal lorsque l'individu est dans une posture naturelle et regarde à l'horizon.

En variante encore, on peut prévoir le paramètre de direction du regard consiste directement en des coordonnées de la droite de visée DVD, DVG déterminées par le dispositif électronique et informatique à partir des signaux représentatifs des images des reflets cornéens des deux yeux.

On mesure en outre le paramètre de posture de la tête suivant :

- une distance DD, DG entre la tête ou l'un des yeux OD, OG de l'individu et le dispositif de capture d'image, par exemple plus précisément la distance DD, DG entre le centre de rotation des yeux OD, OG de l'individu et le dispositif de capture d'image.

On peut également prévoir de mesurer au moins l'un des paramètres de posture de la tête suivants :

- un premier angle AVD, AVG d'inclinaison du plan de capture d'image PCI par rapport à un premier plan de référence PREFD1, PREFG1 perpendiculaire à une droite d'observation DOD, DOG reliant l'un des yeux OD, OG de l'individu, et la pupille 11 de l'appareil de capture d'image 10, autour d'un axe horizontal X' de ce premier plan de ce référence PREFD, PREFG, et
- un deuxième angle AHD, AHG d'inclinaison du plan de capture d'image PCI par rapport à ce premier plan de référence PREFD1, PREFG1 autour d'un axe Y de ce premier plan de référence PREFD1, PREFG1 perpendiculaire audit axe horizontal X'.

Le premier angle AVD, AVG correspond ainsi à l'angle existant dans un plan perpendiculaire audit axe horizontal X' entre une droite définie par l'intersection entre le plan de capture d'image PCI et ce plan perpendiculaire à l'axe horizontal X' et une droite définie par l'intersection entre le plan de référence PREFD1, PREFG1 et ce plan perpendiculaire à l'axe horizontal X'.

Le deuxième angle AVD, AVG correspond ainsi à l'angle existant dans un plan perpendiculaire audit axe Y entre une droite définie par l'intersection entre le plan de capture d'image PCI et ce plan perpendiculaire à l'axe Y et une droite définie par l'intersection entre le plan de référence PREFD1, PREFG1 et ce plan perpendiculaire à l'axe Y.

Pour la mesure de ces paramètres de direction du regard H et de posture de la tête de l'individu, la tête de l'individu est par exemple équipée d'un système de repérage tel que celui décrit dans le document WO2008/132356. Ce système de repérage peut être maintenu directement sur la tête du porteur ou disposé sur une monture de lunettes ne comportant pas de lentilles ophtalmiques.

Les calculs de différents paramètres de posture et notamment de la distance entre la tête du porteur et l'appareil de capture d'image sont décrit dans ce document.

Le plan anatomique PFH est avantageusement le plan de Francfort décrit dans ce document. Le plan anatomique incliné PFI correspond à ce plan de Francfort lorsque la tête de l'individu est en posture naturelle et dirige son regard en direction de l'appareil de capture d'image et non à l'infini.

En particulier, on réalise une étape préalable de calibrage lors de laquelle on détermine la position du plan de Francfort lorsque l'individu regarde au loin. Ce plan de Francfort est alors sensiblement horizontal. Il est alors possible de déduire la position du plan de Francfort lorsque la tête de l'individu est inclinée à partir des caractéristiques géométriques de l'image d'un élément de repérage vertical du système de repérage décrit dans ce document. Il s'agit par exemple de l'écart entre des bandes sombres d'un motif représenté sur cet élément de repérage vertical,

Ce document décrit en outre une méthode de détermination de la position des centres de rotation des yeux de l'individu dans le référentiel de sa tête. Cette détermination peut également être réalisée à titre d'étape de calibrage.

Lorsque l'individu fixe la pupille de l'appareil de capture d'image, la position des centres de rotation des yeux ayant été déterminée dans le référentiel de la tête et la position de la tête ayant été déterminée dans le référentiel de capture d'image, on en déduit la position des centres de rotation des yeux dans le référentiel de capture d'image. Si l'individu regarde la pupille de l'appareil de capture d'image, on peut donc en déduire la direction de regard des yeux de l'individu ainsi que la position de la pupille de l'appareil de capture d'image par rapport au plan de Francfort incliné.

En variante, on pourra également utiliser une méthode d'estimation de la posture de la tête du porteur en trois dimensions selon la méthode décrite dans le document « Real-time modeling of face déformation for 3D head pose estimation » publié par M. Oka et al. dans le livre « Analysis and Modelling of Faces and Gestures » paru aux éditions Springer en 2005, aux pages 308-à 320.

On peut également envisager d'utiliser toute méthode « d'eye tracking » connue de l'homme du métier pour déterminer le paramètre de direction du regard, telle que la méthode décrite dans le document US2003/0169907.

Typiquement, dans une méthode d'«eye tracking», c'est-à-dire de suivi du regard, une image est traitée pour trouver les coordonnées des reflets de la source lumineuse sur la rétine de chaque oeil de l'individu. Cette étape est réalisée par exemple par convolution de l'image par le motif théorique du reflet. Les coordonnées des reflets sont par exemple déterminées en termes de coordonnées des pixels de l'image représentant ce reflet.

Le bord de la pupille est détecté par exemple par détection des contours par gradient et transformée de Hough, ce qui permet de déterminer les coordonnées du centre de la pupille et son diamètre.

Une étape de calibration permet en outre de déterminer la loi, par exemple polynomiale ou autre, qui convertit la position du reflet de la source et les coordonnées du centre de la pupille en angle de la direction de regard. Pour cela, au cours de cette étape de calibration l'individu fixe du regard des points de visée dont la position en trois dimensions, ou au moins la position angulaire dans le référentiel de capture d'image est connue.

Une autre méthode de calibration consisterait à calculer la position de la pupille en deux dimensions dans un plan parallèle au référentiel attaché à la tête de l'individu. En particulier, ce plan parallèle au référentiel attaché à la tête de l'individu peut être un plan frontal perpendiculaire audit plan de Francfort.

Avantageusement, le dispositif électronique et informatique met aussi en mémoire les paramètres de posture de la tête mesurés en plus du paramètre de direction du regard. La caractéristique de réfraction peut ainsi être calculée et éventuellement ultérieurement corrigée en fonction de ces paramètres de posture de la tête.

Par exemple, dans le cas où la droite de visée DVD, DVG suivant la direction du regard de l'individu fait un angle theta avec la droite d'observation DOD, DOG reliant l'oeil de l'individu et la pupille de l'appareil de capture d'image, l'image de la pupille de l'individu sur l'image capturée n'est pas circulaire, mais elliptique. Le calculateur est alors programmé pour diviser le plus petit diamètre de l'ellipse par un facteur égal au cosinus de cet angle theta afin de reconstruire la forme réelle de la pupille, et donc du reflet de l'image de la source lumineuse. Cela permet de déterminer plus précisément la caractéristique de réfraction issue du traitement de l'image correspondante.

En pratique, à l'étape e'), le dispositif électronique et informatique compare le sens du déplacement de l'image du reflet rétinien de la source sur deux images correspondant à des positions différentes de la source lumineuse et le sens de déplacement de la source elle-même entre les deux captures d'image correspondantes.

Dans l'exemple représenté sur les figures 2 à 5, la source se déplace du lieu de la diode 21 au lieu de la diode 22, c'est-à-dire de la gauche vers la droite dans le référentiel de la tête du porteur, selon un axe sensiblement horizontal dans le référentiel de la tête de l'individu.

L'image du reflet rétinien IROD2, IROG2 de la diode 22 imagée à travers la pupille de chacun des yeux de l'individu se trouve elle décalée légèrement sur la gauche dans ce référentiel par rapport à l'image du reflet rétinien IROD1, IROG1 de la diode 21.

L'image du reflet rétinien IROD1, IROG1 s'est donc déplacée dans un sens opposé à celui du déplacement de la source lumineuse, selon un axe de l'oeil parallèle à l'axe de déplacement de la diode allumée. Dans cet exemple, ces axes sont sensiblement horizontaux.

Conformément au principe de la skiascopie, le dispositif électronique et informatique est programmé pour déduire de cette comparaison que les yeux OD, OG de l'individu présente une amétropie négative, c'est-à-dire une myopie, selon un méridien de l'oeil perpendiculaire à l'axe de l'oeil parallèle à l'axe de déplacement de la diode allumée, c'est-à-dire ici selon le méridien vertical.

Dans le cas où le déplacement de l'image du reflet se fait dans le même sens que le déplacement de la source lumineuse, le dispositif électronique et informatique est programmé pour déduire de cette comparaison que les yeux OD, OG de l'individu présentent une amétropie positive, c'est-à-dire une hypermétropie selon le méridien correspondant.

Ici, la disposition des diodes 20 dans le plan du dispositif de mesure autorise la détermination du signe de l'amétropie des deux yeux selon quatre méridiens de l'oeil, dont deux méridiens orientés à 45 degrés d'un premier méridien et un méridien orienté à 90 degrés de ce premier méridien, en allumant successivement au moins deux diodes disposées selon chacune des quatre directions d'alignement des diodes sur la face avant du support 30.

En variante, dans une étape e) le dispositif électronique et informatique détermine, à partir du signal représentatif d'au moins une image, et de préférence à partir des signaux représentatifs de chaque image, la largeur de l'image du reflet rétinien IROD1, IROD2, IROG1, IROG2 de la source lumineuse et en déduit une valeur de l'amétropie des yeux de l'individu.

Dans l'exemple décrit ici, les sources lumineuses étant des diodes, elle présentent une forme circulaire.

Dans le cas d'un oeil ne présentant pas d'astigmatisme, l'image du reflet rétinien de la source présente alors une forme circulaire. La largeur de l'image du reflet rétinien correspond alors au diamètre du reflet.

Dans le cas d'un oeil présentant un astigmatisme, l'image du reflet rétinien présente une forme elliptique. L'axe de cette ellipse présentant le plus grand diamètre correspond alors au méridien de l'oeil présentant la plus forte amétropie en valeur absolue. La largeur de l'image du reflet rétinien correspond alors au plus grand diamètre de cette ellipse.

Pour déterminer cette largeur, le dispositif électronique et informatique analyse sur chaque image capturée la répartition spatiale d'intensité du reflet rétinien de la source correspondante.

Cette répartition spatiale d'intensité est représentée schématiquement sur la figure 8. Chaque courbe de la figure 8 correspond à une proportion de lumière réfléchie par l'oeil.

Les points situés sur le plateau de chaque courbe correspondent au cas d'un reflet recouvrant toute la surface de la pupille, tandis que les points situés sur les pentes de chaque courbe correspondent à un reflet dont le bord est visible dans la pupille de l'oeil, comme représenté sur les figures 3 et 5. La répartition spatiale de l'intensité est ici représentée pour différentes valeurs de l'amétropie D exprimées en dioptrie dans la légende.

Ici, la répartition spatiale d'intensité est par exemple égale à un pourcentage de surface de la pupille éclairée par le reflet de la source lumineuse.

Pour déterminer ce pourcentage, le dispositif électronique et informatique détermine la forme de la pupille de l'oeil et le lieu de la limite entre la zone de l'image de la pupille de l'oeil éclairée par le reflet de la source lumineuse, et la zone sombre de la pupille de l'oeil de l'individu. La détermination de ce pourcentage est d'autant plus précise que la forme de la pupille de l'oeil est connue précisément, comme mentionné précédemment.

Le dispositif électronique et informatique détermine alors le diamètre du reflet rétinien correspondant à chaque répartition d'intensité qui est par exemple égale à la largeur à mi-hauteur de cette répartition spatiale d'intensité.

Si la forme du reflet rétinien est elliptique, le dispositif électronique et informatique détermine la répartition spatiale d'intensité le long du plus grand axe de l'ellipse et en déduit la largeur du reflet rétinien.

En pratique, la qualité de l'oeil réel et les dimensions de la source peuvent faire varier la répartition spatiale d'intensité observée sur les images capturées, et notamment étaler cette répartition. Le dispositif électronique et informatique peut avantageusement être programmé pour corriger ces variations, notamment grâce à une étape de calibrage. Cette étape de calibrage est par exemple réalisée à l'aide d'un oeil artificiel présentant une aberration connue, de manière bien connue de l'homme du métier.

Avantageusement, le dispositif électronique et informatique reconstitue la répartition spatiale d'intensité du reflet à partir de plusieurs images capturées, montrant chacune une partie de cette répartition. On peut prévoir notamment que la série d'images capturées permette de suivre le déplacement de l'image du reflet rétinien de la source de manière à le visualiser entièrement, et à obtenir la répartition spatiale d'intensité complète du reflet rétinien de la source.

Le dispositif électronique et informatique détermine ainsi très précisément la largeur de l'image du reflet rétinien.

La largeur L de l'image du reflet rétinien de la source vérifie la relation suivante :

$$L = E* (-2*D*DPR+DPR/DPS - 1),$$

où E est le diamètre de la pupille de l'oeil de l'individu considéré,
DPR est la distance entre la pupille de l'appareil de capture d'image et la rétine de l'oeil de l'individu,
DSR est la distance entre la source lumineuse et la rétine de l'oeil de l'individu.
D est la valeur en dioptrie de l'amétropie de l'oeil.

La valeur D de l'amétropie peut donc être calculée par la formule suivante:

$$D = (L/E - DPR/DPS + 1)/(-2DPR).$$

Le diamètre de la pupille de l'oeil de l'individu peut être soit rentré par l'utilisateur comme paramètre de calcul dans le dispositif électronique et informatique, soit mesuré à partir des images capturées, par exemple grâce au traitement de l'image décrit précédemment dans la méthode d'« eye tracking ».

Dans le premier cas, l'utilisateur rentre une valeur moyenne de la taille de la pupille prédéterminée. Dans le deuxième cas, le système électronique de traitement de l'image identifie la pupille d'au moins un oeil de l'individu sur au moins une image d'une série d'image enregistrée, et mesure son diamètre. Il en déduit la taille réelle de la pupille en multipliant le diamètre ainsi mesuré par un facteur de mise à l'échelle. Ce facteur de mise à l'échelle est déterminé par exemple grâce au système de repérage et à la méthode décrits dans le document WO2008/132356.

La distance DPR entre la pupille de l'appareil de capture d'image et la rétine de l'oeil de l'individu est déterminée à partir des paramètres de posture mesurés à l'étape c). Ces paramètres de posture comportent notamment la distance DD, DG entre le centre de rotation des yeux OD, OG de l'individu et le dispositif de capture d'image.

La distance entre la rétine et le centre de rotation de l'oeil étant égale environ à 11 millimètres, la distance DPR entre la pupille de l'appareil de capture d'image et la rétine des yeux de l'individu est égale à la distance DD, DG entre le centre de rotation des yeux OD, OG de l'individu et le dispositif de capture d'image à laquelle on ajoute 11 millimètres.

La distance DSR entre la source lumineuse et la rétine de l'oeil de l'individu est calculée par le dispositif électronique et informatique à partir de la distance DPR entre la pupille de l'appareil de capture d'image et la rétine des yeux de l'individu et de la position de la source lumineuse allumées lors de la capture d'image par rapport à la pupille de l'appareil de capture d'image qui est connue.

Dans l'exemple décrit ici, les distances DPR et DSR sont très proches et on peut considérer en première approximation qu'elles sont égales.

La valeur de l'amétropie c'est-à-dire la puissance sphérique ou cylindrique de l'oeil est ainsi calculée par la formule précédente.

Afin d'accélérer la mise en oeuvre du procédé selon l'invention, il est avantageux de réaliser les captures d'image des yeux de l'individu qui donnent les informations les plus complète sur la taille et la forme du reflet rétinien de la source allumée, c'est-à-dire qui permettent de visualiser au mieux les bords de ce reflet rétinien.

On réalise par exemple la capture d'une première série d'images en allumant successivement les diodes situées sur une même direction d'alignement, selon une séquence alternant les diodes situées de chaque côté de la pupille 11 de l'appareil de capture d'image 10, en commençant par les diodes disposées au plus près de cette pupille 11 et en s'éloignant progressivement de celle-ci. Le pas entre deux diodes allumées successivement du même côté double par exemple à chaque itération, de manière à balayer rapidement toutes les diodes correspondant à la direction d'alignement considérée.

On réalise ensuite les captures d'images suivantes en allumant uniquement les diodes pour lesquelles l'intensité du reflet est égale en moyenne à 50% et à 90% de l'intensité maximale du reflet, Lorsque l'intensité du reflet est égale à 50% de l'intensité maximale du reflet, le bord du reflet, c'est-à-dire la limite entre la zone de la pupille de l'oeil éclairée par le reflet et la zone de la pupille de l'oeil non éclairée partage la pupille de l'oeil en deux zones de surfaces égales. Cela correspond à l'allumage de quatre diodes alignées de part et d'autre de la pupille de l'appareil de capture d'image.

La détermination de la largeur du reflet est ainsi plus précise.

Afin de déterminer l'astigmatisme de l'oeil, le dispositif électronique et informatique analyse la forme du reflet, en déterminant les répartitions spatiales d'intensité du reflet, et donc la valeur de l'amétropie selon différents méridiens de l'oeil, par exemple selon trois méridiens choisis arbitrairement et disposés relativement les uns par rapport aux autres à 0, 45 et 90 degrés.

Le dispositif électronique et informatique réalise ensuite d'autres mesures selon les méridiens de l'oeil correspondant aux plus petite et plus grande valeurs de l'amétropie, c'est-à-dire qu'il détermine le plus petit et le plus grand diamètre de l'ellipse. Il en déduit des valeurs précises de l'amétropie selon les deux méridiens correspondant car le diamètre de l'ellipse varie peu autour du plus grand et du plus petit axe de l'ellipse.

Enfin, il détermine les diamètres de l'ellipse à 45 degrés de part et d'autre du grand axe de l'ellipse. Cette dernière mesure permet de déterminer précisément l'axe de l'astigmatisme car la valeur du diamètre de l'ellipse varie rapidement autour de l'axe situé à 45 degrés du plus grand axe. L'astigmatisme est ainsi déterminé très précisément.

Dans ce qui précède, l'individu n'est pas équipé d'une monture de lunettes comportant des lentilles ophtalmiques correctrices. La valeur de la caractéristique de réfraction obtenue est donc celle des yeux de l'individu eux-mêmes. En variante, on peut envisager que l'opérateur place sur le visage dudit individu un équipement de correction visuelle, comportant par exemple des lentilles ophtalmiques de puissance et de déviation connues.

Dans ce cas, le dispositif électronique et informatique est programmé pour corriger les signaux représentatifs des images capturées et/ou la caractéristique de réfraction déduite de ces signaux en fonction de la puissance et de la déviation de l'équipement de correction visuelle.

Il s'agit par exemple dans le cas simple d'une lentille de correction visuelle sphérique de puissance donnée de rajouter cette puissance à la valeur de l'amétropie déterminée par le procédé décrit précédemment. La somme de ces deux valeurs donne la caractéristique de réfraction des yeux de l'individu.

On peut alors également appliquer à nouveau le procédé selon l'invention pour déterminer une sous-correction ou une sur-correction des yeux de l'individu.

Bien entendu, l'individu peut également être équipé d'une monture de lunettes ne comprenant pas de lentilles de correction visuelles. Cette monture sert par exemple de support au système de repérage utilisé pour déterminer le ou les paramètres de posture de la tête de l'individu.

De préférence, la direction du regard de l'individu dans le référentiel de l'appareil de capture d'image reste la même lors des captures d'image. Pour cela, le dispositif électronique et informatique déclenche les captures d'images à des intervalles très courts, par exemple à 100 millisecondes d'intervalle, de sorte que les yeux de l'individu bougent peu entre deux captures d'image.

La présente invention n'est nullement limitée aux modes de réalisation décrits et représentés mais l'homme du métier saura y apporter toute variante conforme à son esprit.

En particulier, le dispositif de mesure peut comporter en variante un dispositif de mesure à ultra-son, comprenant au moins un émetteur et un récepteur d'ultra-son ou un dispositif de mesure à capteurs électromagnétiques pour mesurer des paramètres de posture de la tête de l'individu.

En variante, à l'étape e), on détermine à partir de ladite au moins une image capturée, une répartition de luminance de l'image du reflet rétinien IROD1, IROG1, IROD2, IROG2 de la source lumineuse 20 et on détermine ladite caractéristique de réfraction D des yeux OD, OG de l'individu en fonction de cette répartition de luminance.

La caractéristique de réfraction est alors déterminée par une méthode classique de photoréfraction telle que décrite dans l'article publié dans « Optometry and vision science », volume 174, pages 472 à 482 en 1997. La caractéristique de réfraction de l'oeil, ici le signe et/ou la valeur de l'amétropie est déterminée à partir de la répartition de luminance de l'image du reflet. Ceci est réalisé grâce à une étape préalable de calibrage.

Les différents modes de réalisation et leurs variantes décrites en référence à la méthode de calcul de la caractéristique de réfraction par skiascopie s'appliquent de manière similaire au procédé utilisant une méthode de calcul de la caractéristique de réfraction par photoréfraction.

Par exemple, on peut envisager de mettre en oeuvre le procédé selon l'invention en capturant au moins deux images du reflet rétinien de ladite source lumineuse sur la rétine des deux yeux de l'individu, lors de ladite étape de capture d'image, et en déterminant à partir de l'une de ces deux images, ladite caractéristique de réfraction de l'un des deux yeux de l'individu et à partir de l'autre image, la caractéristique de réfraction de l'autre oeil de l'individu.

Selon une autre variante, le procédé selon l'invention comporte un test psychophysique réalisé selon les étapes suivantes:

r) afficher devant les yeux de l'individu une image présentant un flou correspondant à un filtrage passe-bas par un filtre de fréquence de coupure donnée des fréquences spatiales de cette image,

t) mettre en mémoire la caractéristique de réfraction déterminée en relation avec le résultat du test psychophysique réalisé à l'étape s) et la valeur de la fréquence de coupure du filtre passe-bas utilisé à l'étape r),

u) réitérer au moins les étapes b), c) ainsi que les étapes de capture d'image de de détermination d'une caractéristique de réfraction et les étapes r) et t) pour différentes fréquences de coupure du filtre de l'image.

De préférence, le test psychophysique comporte en outre une étape s) de caractérisation de la perception du flou de l'image par l'individu, et à l'étape u), le résultat de ce test est également mis en mémoire en relation avec la fréquence de coupure du filtre correspondante.

Le dispositif de mesure 100 comporte à cet effet des moyens de génération d'une image présentant un flou d'affichage et un dispositif d'affichage de cette image. Ces moyens d'affichage sont par exemple constitués d'un écran intégré dans le boîtier du dispositif décrit précédemment et adapté à afficher ladite image floue.

L'image floue est par exemple générée par le dispositif électronique et informatique. Elle est obtenue par filtrage spatiale d'une partie des fréquences spatiales d'une image nette, par exemple d'une image représentant un caractère alphanumérique. Le dispositif électronique et informatique détermine l'image floue en appliquant numériquement un filtre passe-bas de fréquence de coupure prédéterminée à l'image nette.

On peut également envisager que l'image floue soit obtenue par un filtrage analogique des signaux d'affichage de l'écran.

De manière générale, cette image floue peut être obtenue en variante par toute méthode connue de l'homme du métier, notamment par tout traitement d'image permettant l'obtention d'une image floue.

L'image nette peut être décomposée en plusieurs composantes de fréquences spatiales différentes.

Au cours du filtrage, les amplitudes des composantes présentant une fréquence spatiale élevée sont atténuées. Les contours du caractère alphanumérique représenté sur l'image apparaissent alors diffus.

Le filtre passe-bas utilisé est caractérisé par une fréquence spatiale de coupure correspondant à un seuil au-delà duquel les fréquences spatiales de l'image sont plus fortement atténuées que les fréquences spatiales de l'image inférieures audit seuil. La fréquence spatiale de coupure correspond alors à la distance minimale entre deux détails de l'image floue pouvant être distingués.

Cette image floue peut être calculée en temps réel ou enregistrée dans une bibliothèque d'image du dispositif électronique et informatique.

Le dispositif électronique et informatique communique avec l'écran pour lui faire afficher l'image floue calculée.

L'étape s) de caractérisation de la perception du flou peut consister par exemple à interroger l'individu sur sa perception du flou de l'image affichée. Les questions posées visent par exemple à mettre en évidence la détection du flou de l'image par l'individu, une gêne ressentie par l'individu en raison du flou et/ou une perte de lisibilité du caractère alphanumérique représenté sur l'image floue.

Les réponses de l'individu aux questions posées caractérisent sa perception du flou pour le degré de flou de l'image affichée. Elles sont mises en mémoire dans le dispositif électronique et informatique en relation avec la caractéristique de réfraction déterminée et la valeur de la fréquence de coupure du filtre passe-bas utilisé à l'étape r).

En outre, selon le procédé conforme à l'invention, on réitère au moins les étapes b), c) ainsi que les étapes de capture d'image de détermination d'une caractéristique de réfraction et les étapes r) à t) pour différentes fréquences de coupure du filtre de l'image.

Il est alors avantageusement possible de déterminer l'évolution de la perception du flou et de la caractéristique de réfraction des yeux de l'individu lorsque le degré de flou de l'image varie.

On a décrit en détail un mode de réalisation de l'invention dans lequel les caractéristiques de réfraction des deux yeux sont déterminées à partir d'au moins une même image, par exemple à partir de la même série d'images.

En variante, on peut envisager que :

- lors de ladite étape de capture d'image, on capture successivement au moins deux images comportant chacune les images des deux reflets rétiniens de ladite source lumineuse sur les rétines des deux yeux de l'individu

- on détermine, à partir de l'une de ces deux images, ladite caractéristique de réfraction de l'un des deux yeux de l'individu et à partir de l'autre image, la caractéristique de réfraction de l'autre oeil de l'individu.

Par exemple, il est possible d'envisager que l'on capture une série d'images et que l'on traite une partie de ces images pour déterminer la caractéristique de réfraction d'un oeil et une autre partie de ces images pour déterminer la caractéristique de réfraction de l'autre oeil. Le reste du procédé selon l'invention reste inchangé.

Dans ce cas, il est possible d'affiner le choix des images traitées pour chaque oeil indépendamment, ce qui permet par exemple de choisir précisément pour chaque oeil les images capturées pour lesquelles l'intensité du reflet est égale en moyenne à 50% et à 90% de l'intensité maximale du reflet,

Ceci est notamment utile lorsque les deux yeux présentent des amétropies très différentes.

## Revendications

1. Procédé de mesure automatique d'au moins une caractéristique de réfraction des deux yeux (OD, OG) d'un individu, comportant les étapes suivantes:

   a) ajuster au moins une posture de la tête de l'individu selon le souhait dudit individu, dans un référentiel (O, X, Y, Z) lié à un appareil de capture d'image (10) adapté à convertir chaque image capturée en un signal qui en est représentatif,
   b) éclairer les deux yeux (OD, OG) au moyen d'au moins une source lumineuse (20) dont la position, dans ce référentiel (O, X, Y, Z) lié à l'appareil de capture d'image (10), est connue,
   c) mesurer au moins un paramètre de direction de regard lié à la direction du regard de l'individu dans un référentiel lié à sa tête, ce paramètre de direction de regard consistant exclusivement en un paramètre de posture (DD, DG, H, AHD, AHG, AVD, AVG) de la tête de l'individu, ce paramètre de posture étant mesuré lorsque l'individu fixe du regard une pupille de l'appareil de capture d'image et représentatif de l'abaissement du regard vers la pupille de l'appareil de capture d'image, ce paramètre de posture (DD, DG, H, AHD, AHG, AVD, AVG) de la tête comprenant au moins l'un des paramètres suivants :

      - une distance (DD, DG) entre la tête ou l'un des yeux (OD, OG) de l'individu et le dispositif de capture d'image,
      - une distance verticale (H) entre une pupille de l'appareil de capture d'image (10) et un plan anatomique incliné (PFI) lorsque l'individu est dans une posture naturelle et dirige son regard en direction de l'appareil de capture d'image, correspondant à un plan anatomique d'horizon (PFH) qui est sensiblement horizontal lorsque l'individu est dans une posture naturelle et regarde à l'horizon,

- une distance entre une pupille de l'appareil de capture d'image (10) et le plan anatomique d'horizon (PFH) sensiblement horizontal lorsque l'individu est dans une posture naturelle et regarde à l'horizon,

- un premier angle d'inclinaison (AVD, AVG) du plan de capture d'image (PCI) par rapport à un premier pian de référence (PREFD1, PREFG1) perpendiculaire à la droite reliant l'un des yeux (OD, OG) de l'individu et la pupille de l'appareil de capture d'image (10), autour d'un axe horizontal (X') de ce premier plan de référence, et

- un deuxième angle d'inclinaison (AHD, AHG) du plan de capture d'image (PCI) par rapport à ce premier plan de référence (PREFD1, PREFG1) autour d'un axe de ce premier plan de référence (PREFD1, PREFG1) perpendiculaire audit axe horizontal (X'),

d) capturer, au moyen dudit appareil de capture d'image (10), au moins une image du reflet rétinien (IROD1, IROG1, IROD2, IROG2) de ladite source lumineuse (20) sur la rétine des deux yeux (OD, OG) de l'individu, correspondant au paramètre de direction du regard mesuré à l'étape c),

f) déterminer, à partir de ladite au moins une image capturée à l'étape d), ladite caractéristique de réfraction (D) des yeux (OD, OG) de l'individu et

h) mettre en mémoire le paramètre de direction de regard lié à la direction du regard de l'individu mesuré lors de la capture d'image de l'étape d) en relation avec la caractéristique de réfraction déduite des images capturées dans les conditions visuelles correspondant à cette direction du regard.

2. Procédé de mesure automatique d'au moins une caractéristique de réfraction des deux yeux (OD, OG) d'un individu dans des conditions visuelles prédéfinies, comportant les étapes suivantes :

a) ajuster au moins une posture de la tête de l'individu selon le souhait dudit individu, dans un référentiel (O, X, Y, Z) lié à un appareil de capture d'image (10) adapté à convertir chaque image capturée en un signal qui en est représentatif,

b) éclairer les deux yeux (OD, OG) au moyen d'au moins une source lumineuse (20) dont la position, dans ce référentiel (O, X, Y, Z) lié à l'appareil de capture d'image (10), est connue,

c) mesurer au moins un paramètre de direction du regard lié à la direction du regard de l'individu dans un référentiel lié à sa tête, ce paramètre de direction du regard consistant exclusivement en un paramètre de posture (DD, DG, H, AHD, AHG, AVD, AVG) de la tête de l'individu, ce paramètre de posture étant mesuré lorsque l'individu fixe du regard une pupille de l'appareil de capture d'image et représentatif de l'abaissement du regard vers la pupille de l'appareil de capture d'image, ce paramètre de posture (DD, DG, H, AHD, AHG, AVD, AVG) de la tête comprenant au moins l'un des paramètres suivants :

- une distance (DD, DG) entre la tête ou l'un des yeux (OD, OG) de l'individu et le dispositif de capture d'image,
- une distance verticale (H) entre une pupille de l'appareil de capture d'image (10) et un plan anatomique incliné (PFI) lorsque l'individu est dans une posture naturelle et dirige son regard en direction de l'appareil de capture d'image, correspondant à un pian anatomique d'horizon (PFH) qui est sensiblement horizontal lorsque l'individu est dans une posture naturelle et regarde à l'horizon,
- une distance entre une pupille de l'appareil de capture d'image (10) et le plan anatomique d'horizon (PFH) sensiblement horizontal lorsque l'individu est dans une posture naturelle et regarde à l'horizon,
- un premier angle d'inclinaison (AVD, AVG) du plan de capture d'image (PCI) par rapport à un premier plan de référence (PREFD1, PREFG1) perpendiculaire à la droite reliant l'un de yeux (OD, OG) de l'individu et la pupille de l'appareil de capture d'image (10), autour d'un axe horizontal (X') de ce premier plan de référence, et
- un deuxième angle d'inclinaison (AHD, AHG) du plan de capture d'image (PCI) par rapport à ce premier pian de référence (PREFD1, PREFG1) autour d'un axe de ce premier plan de référence (PREFD1, PREFG1) perpendiculaire audit axe horizontal (X'),

i) comparer le paramètre de direction du regard mesuré à l'étape c) avec un intervalle de valeur correspondant aux conditions visuelles prédéfinies et déclencher la capture, au moyen dudit appareil de capture d'image (10), d'au moins une image du reflet rétinien (IROD1, IROG1, IROD2, IROG2) de ladite source lumineuse (20) sur la rétine des deux yeux (OD, OG) de l'individu, cette image étant capturée dans un plan de capture d'image (PCI), si la valeur dudit paramètre de direction du regard mesurée à l'étape c) est située dans ledit intervalle correspondant auxdites conditions visuelles prédéfinie,

j) déterminer, à partir de ladite au moins une image capturée, ladite caractéristique de réfraction (D) des yeux (OD, OG) de l'individu dans lesdites conditions visuelles prédéfinies.

**3.** Procédé selon l'une des revendications 1 et 2, comportant en outre l'étape suivante :

e) déterminer, à partir de ladite au moins une image capturée une largeur ou une répartition de luminance de l'image du reflet rétinien (IROD1, IROG1, IROD2, IROG2) de la source lumineuse (20) et déterminer ladite caractéristique de réfraction (D) des yeux (OD, OG) de l'individu en fonction de cette largeur ou de cette répartition de luminance.

**4.** Procédé selon la revendication 3, selon lequel les deux yeux (OD, OG) étant éclairés simultanément, les images des reflets rétiniens de la source lumineuse (20) sur la rétine des deux yeux (OD, OG) sont capturées simultanément et à l'étape e), la largeur ou la répartition de luminance de l'image du reflet rétinien (IROD1, IROG1, IROD2, IROG2) de la source lumineuse (20) est déterminée au moins à partir desdits signaux relatifs aux reflets rétiniens des deux yeux (OD, OG) capturés simultanément dans ladite posture de la tête de l'individu par rapport à ladite source lumineuse (20).

**5.** Procédé selon la revendication 4, selon lequel on déduit une valeur de l'amétropie des yeux (OD, OG) de l'individu de la largeur (L) ou de la répartition de luminance de l'image du reflet rétinien (IROD1, IROG1, IROD2, IROG2) de la source lumineuse (20).

**6.** Procédé selon l'une des revendications 1 et 2, dans lequel, au moins deux images du reflet rétinien (IROD1, IROG1, IROD2, IROG2) de ladite source lumineuse (20) sur la rétine des deux yeux (OD, OG) de l'individu étant capturées, le procédé comporte en outre l'étape suivante :

e') déterminer, à partir desdites au moins deux images capturées un déplacement de l'image du reflet rétinien (IROD1, IROG1, IROD2, IROG2) de la source lumineuse (20),

ladite caractéristique de réfraction (D) des yeux (OD, OG) de l'individu étant déterminée en fonction de ce déplacement.

**7.** Procédé selon la revendication 6, dans lequel

les postures relatives de la tête de l'individu par rapport à ladite source lumineuse (20) étant différentes lors des deux captures d'image,

à l'étape c), on mesure, lors desdites captures d'image, au moins un paramètre de posture (DD, DG, AHD, AHG, AVD, AVG) de la tête de l'individu dans le référentiel (O, X, Y, Z) de l'appareil de capture d'image (10),

à l'étape e'), on compare le déplacement de l'image du reflet rétinien déterminé à la différence des postures relatives de la tête de l'individu par rapport à la source lumineuse (20) entre les deux captures d'image, en fonction dudit paramètre de posture (DD, DG, AHD, AHG, AVD, AVG) de la tête mesuré à l'étape c),

ladite caractéristique de réfraction (D) des yeux (OD, OG) de l'individu étant déduite de cette comparaison.

**8.** Procédé selon la revendication 7, selon lequel le signe de l'amétropie des yeux (OD, OG) de l'individu est déduit de ladite comparaison.

**9.** Procédé selon l'une des revendications 6 à 8, selon lequel les deux yeux (OD, OG) étant éclairés simultanément, les images des reflets rétiniens de la source lumineuse (20) sur la rétine des deux yeux (OD, OG) sont capturées simultanément et selon lequel, à l'étape e'), le déplacement de l'image du reflet rétinien (IROD1, IROG1, IROD2, IROG2) de la source lumineuse (20) est déterminé au moins à partir desdits signaux relatifs aux reflets rétiniens des deux yeux (OD, OG) capturés simultanément dans chaque posture de la tête de l'individu par rapport à ladite source lumineuse (20).

**10.** Procédé selon l'une des revendications 1 à 9, dans lequel, lors de la capture d'image, un support (30) sur lequel sont montés ladite source lumineuse (20) et ledit appareil de capture d'image (10) est porté à la main par ledit individu.

**11.** Procédé selon l'une des revendications 1 à 10, comportant une étape de vérification antérieure ou postérieure à l'étape de capture d'image, de ce que ladite au moins une image est capturée lorsque l'individu regarde au voisinage, à l'intérieur d'une plage déterminée, d'une pupille (11) de l'appareil de capture d'image (10).

**12.** Procédé selon l'une des revendications 3 à 11, selon lequel, lors d'une étape préliminaire à la capture d'au moins une image, on place sur le visage dudit individu un équipement de correction visuelle et selon lequel on corrige les signaux représentatifs de ladite image capturée et/ou la caractéristique de réfraction déduite de ces signaux en fonction de la puissance et de la déviation de l'équipement de correction visuelle.

**13.** Procédé selon l'une des revendications 1 à 12, dans lequel, à l'étape c), on calcule la direction (DVD, DVG) du regard de l'individu à partir des signaux représentatifs des images des reflets cornéens des deux yeux (OD, OG).

**14.** Procédé selon l'une des revendications précédentes, comportant les étapes suivantes:

r) avant ou pendant la capture d'au moins une image, afficher devant les yeux de l'individu une image de référence présentant un flou correspondant à un filtrage passe-bas par un filtre de fréquence de coupure donnée des fréquences spatiales de cette image de référence,
t) mettre en mémoire la caractéristique de réfraction déterminée en relation avec la valeur de la fréquence de coupure du filtre passe-bas utilisé à l'étape r) pour filtrer l'image de référence,
u) réitérer au moins les étapes b), c), d), f), r) et t) ou les étapes b), c), i), j), r) et t) pour différentes fréquences de coupure du filtre de l'image de référence.

**15.** Procédé selon l'une des revendications 1 et 2, selon lequel

- lors de ladite étape de capture d'image, on capture successivement au moins deux images comportant chacune les images des deux reflets rétiniens de ladite source lumineuse sur les rétines des deux yeux de l'individu,
- on détermine, à partir de l'une de ces deux images, ladite caractéristique de réfraction de l'un des deux yeux de l'individu et à partir de l'autre image, la caractéristique de réfraction de l'autre oeil de l'individu.

**16.** Dispositif de mesure automatique d'au moins une caractéristique de réfraction des deux yeux (OD, OG) d'un individu, comportant :

- un support (30),
- au moins une source lumineuse (20) montée sur ce support (30) de manière à éclairer au moins l'un des yeux (OD, OG) de l'individu,
- au moins un appareil de capture d'image (10) monté sur ce support (30), adapté à capturer au moins une image du reflet rétinien (IROD1, IROG1, IROD2, IROG2) de ladite source lumineuse (20) sur la rétine de chaque oeil de l'individu et à convertir chaque image capturée en un signal qui en est représentatif, la position de ladite source lumineuse (20) dans un référentiel (O, X, Y, Z) lié à cet appareil de capture d'image (10) étant connue,

- des moyens de mesure d'au moins un paramètre de direction du regard lié à la direction du regard de l'individu dans un référentiel lié à sa tête, consistant exclusivement en un paramètre de posture (DD, DG, H, AHD, AHG, AVD, AVG) de la tête de l'individu mesuré lorsque l'individu fixe du regard une pupille de l'appareil de capture d'image, représentatif de l'abaissement du regard vers la pupille de l'appareil de capture d'image, ce paramètre de direction de regard correspondant à des conditions visuelles, et le paramètre de posture de la tête (DD, DG, H, AHD, AHG, AVD, AVG) comprenant au moins l'un des paramètres suivants :

- une distance (DD, DG) entre la tête ou l'un des yeux (OD, OG) de l'individu et le dispositif de capture d'image,
- une distance verticale (H) entre une pupille de l'appareil de capture d'image (10) et un plan anatomique incliné (PFI) lorsque l'individu est dans une posture naturelle et dirige son regard en direction de l'appareil de capture d'image, correspondant à un plan anatomique d'horizon (PFH) qui est sensiblement horizontal lorsque l'individu est dans une posture naturelle et regarde à l'horizon,
- une distance entre une pupille de l'appareil de capture d'image (10) et le plan anatomique d'horizon (PFH) sensiblement horizontal lorsque l'individu est dans une posture naturelle et regarde à l'horizon,
- un premier angle d'inclinaison (AVD, AVG) du plan de capture d'image (PCI) par rapport à un premier plan de référence (PREFD1, PREFG1) perpendiculaire à la droite reliant l'un des yeux (OD, OG) de l'individu et la pupille de l'appareil de capture d'image (10), autour d'un axe horizontal (X') de ce premier plan de référence, et
- un deuxième angle d'inclinaison (AHD, AHG) du plan de capture d'image (PCI) par rapport à ce premier plan de référence (PREFD1, PREFG1) autour d'un axe de ce premier plan de référence (PREFD1, PREFG1) perpendiculaire audit axe horizontal (X').
- un calculateur adapté à déterminer, à partir du signal représentatif de ladite au moins une image capturée, ladite caractéristique de réfraction (D) des yeux (OD, OG) de l'individu et à mettre en mémoire ledit au moins un paramètre de direction du regard lié à la direction du regard de l'individu en relation avec la caractéristique de réfraction déterminée à partir des images capturées dans les conditions visuelles correspondant à cette direction du regard.

## Patentansprüche

1. Verfahren zur automatischen Messung mindestens einer Brechungscharakteristik der beiden Augen (OD, OG) einer Person, aufweisend die folgenden Schritte:

a) Anpassen mindestens einer Haltung des Kopfes der Person gemäß dem Wunsch der Person in einem Bezugssystem (O, X, Y, Z), das mit einer Bilderfassungsvorrichtung (10) verbunden ist, die geeignet ist, jedes erfasste Bild in ein Signal umzuwandeln, das dafür repräsentativ ist,
b) Beleuchten der beiden Augen (OD, OG) mittels mindestens einer Lichtquelle (20), deren Position in diesem Bezugssystem (O, X, Y, Z), das mit der Bilderfassungsvorrichtung (10) verbunden ist, bekannt ist,
c) Messen mindestens eines Blickrichtungsparameters, der mit der Blickrichtung der Person in einem Bezugssystem verbunden ist, das mit deren Kopf verbunden ist, wobei dieser Blickrichtungsparameter ausschließlich aus einem Haltungsparameter (DD, DG, H, AHD, AHG, AVD, AVG) des Kopfes der Person besteht, wobei dieser Haltungsparameter gemessen wird, wenn die Person eine Pupille der Bilderfassungsvorrichtung mit dem Blick fixiert, und für das Absenken des Blicks zu der Pupille der Bilderfassungsvorrichtung repräsentativ ist, wobei dieser Haltungsparameter (DD, DG, H, AHD, AHG, AVD, AVG) des Kopfes mindestens einen der folgenden Parameter umfasst:

- einen Abstand (DD, DG) zwischen dem Kopf oder einem der Augen (OD, OG) der Person und der Bilderfassungseinrichtung,
- einen vertikalen Abstand (H) zwischen der Pupille der Bilderfassungsvorrichtung (10) und einer geneigten anatomische Ebene (PFI), wenn sich die Person in einer natürlichen Haltung befindet und ihren Blick in Richtung der Bilderfassungsvorrichtung lenkt, die einer anatomischen Horizontebene (PFH) entspricht, die im Wesentlichen horizontal verläuft, wenn sich die Person in einer natürlichen Haltung befindet und zum Horizont schaut,
- einen Abstand zwischen einer Pupille der Bilderfassungsvorrichtung (10) und der anatomischen Horizontebene (PFH), die im Wesentlichen horizontal verläuft, wenn sich die Person in einer natürlichen Haltung befindet und zum Horizont schaut,

- einen ersten Neigungswinkel (AVD, AVG) der Bilderfassungsebene (PCI) bezogen auf eine erste Bezugsebene (PREFD1, PREFG1), die senkrecht zu der Geraden verläuft, die eins der Augen (OD, OG) der Person und die Pupille der Bilderfassungsvorrichtung (10) verbindet, um eine horizontale Achse (X') dieser ersten Bezugsebene, und
- einen zweiten Neigungswinkel (AHD, AHG) der Bilderfassungsebene (PCI) bezogen auf diese erste Bezugsebene (PREFD1, PREFG1) um eine Achse dieser ersten Bezugsebene (PREFD1, PREFG1), die senkrecht zu der horizontalen Achse (X') verläuft,

d) Erfassen, mittels der Bilderfassungsvorrichtung (10), mindestens eines Bilds der Netzhautreflexion (IROD1, IROG1, IROD2, IROG2) der Lichtquelle (20) auf der Netzhaut der beiden Augen (OD, OG) der Person, das dem Blickrichtungsparameter entspricht, der bei Schritt c) gemessen wird,
f) Bestimmen, ausgehend von dem mindestens einen Bild, das bei Schritt d) erfasst wird, der Brechungscharakteristik (D) der Augen (OD, OG) der Person und
h) Speichern des Blickrichtungsparameters, der mit der Blickrichtung der Person verbunden ist, der bei der Bilderfassung des Schritts d) gemessen wird, in Zusammenhang mit der Brechungscharakteristik, die aus den Bildern abgeleitet wird, die unter den visuellen Bedingungen erfasst werden, die dieser Blickrichtung entsprechen.

2. Verfahren zur automatischen Messung mindestens einer Brechungscharakteristik der beiden Augen (OD, OG) einer Person unter vorgegebenen visuellen Bedingungen, aufweisend die folgenden Schritte:

a) Anpassen mindestens einer Haltung des Kopfes der Person gemäß dem Wunsch der Person in einem Bezugssystem (O, X, Y, Z), das mit einer Bilderfassungsvorrichtung (10) verbunden ist, die geeignet ist, jedes erfasste Bild in ein Signal umzuwandeln, das dafür repräsentativ ist,
b) Beleuchten der beiden Augen (OD, OG) mittels mindestens einer Lichtquelle (20), deren Position in diesem Bezugssystem (O, X, Y, Z), das mit der Bilderfassungsvorrichtung (10) verbunden ist, bekannt ist,
c) Messen mindestens eines Blickrichtungsparameters, der mit der Blickrichtung der Person in einem Bezugssystem verbunden ist, das mit deren Kopf verbunden ist, wobei dieser Blickrichtungsparameter ausschließlich aus einem Haltungsparameter (DD, DG, H, AHD, AHG, AVD, AVG) des Kopfes der Person besteht, wobei dieser Haltungsparameter gemessen wird, wenn die Person eine Pupille der Bilderfassungsvorrichtung mit dem Blick fixiert, und für das Absenken des Blicks zu der Pupille der Bilderfassungsvorrichtung repräsentativ ist, wobei dieser Haltungsparameter (DD, DG, H, AHD, AHG, AVD, AVG) des Kopfes mindestens einen der folgenden Parameter umfasst:

- einen Abstand (DD, DG) zwischen dem Kopf oder einem der Augen (OD, OG) der Person und der Bilderfassungseinrichtung,
- einen vertikalen Abstand (H) zwischen der Pupille der Bilderfassungsvorrichtung und einer geneigten anatomische Ebene (PFI), wenn sich die Person in einer natürlichen Haltung befindet und ihren Blick in Richtung der Bilderfassungsvorrichtung lenkt, die einer anatomischen Horizontebene (PFH) entspricht, die im Wesentlichen horizontal verläuft, wenn sich die Person in einer natürlichen Haltung befindet und zum Horizont schaut,
- einen Abstand zwischen einer Pupille der Bilderfassungsvorrichtung (10) und der anatomischen Horizontebene (PFH), die im Wesentlichen horizontal verläuft, wenn sich die Person in einer natürlichen Haltung befindet und zum Horizont schaut,
- einen ersten Neigungswinkel (AVD, AVG) der Bilderfassungsebene (PCI) bezogen auf eine erste Bezugsebene (PREFD1, PREFG1), die senkrecht zu der Geraden verläuft, die eins der Augen (OD, OG) der Person und die Pupille der Bilderfassungsvorrichtung (10) verbindet, um eine horizontale Achse (X') dieser ersten Bezugsebene, und
- einen zweiten Neigungswinkel (AHD, AHG) der Bilderfassungsebene (PCI) bezogen auf diese erste Bezugsebene (PREFD1, PREFG1) um eine Achse dieser ersten Bezugsebene (PREFD1, PREFG1), die senkrecht zu der horizontalen Achse (X') verläuft,

i) Vergleichen des Blickrichtungsparameters, der bei Schritt c) gemessen wird, mit einem Wertintervall, das den vorgegebenen visuellen Bedingungen entspricht, und Auslösen der Erfassung, mittels der Bilderfassungsvorrichtung (10), mindestens eines Bilds der Netzhautreflexion (IROD1, IROG1, IROD2, IROG2) der Lichtquelle (20) auf der Netzhaut der beiden Augen (OD, OG) der Person, wobei dieses Bild in einer Bilderfassungsebene (PCI) erfasst wird, wenn der Wert des Blickrichtungsparameters, der bei Schritt c) gemessen wird, in dem Intervall liegt, das den vorgegebenen visuellen Bedingungen entspricht,

j) Bestimmen, ausgehend von dem mindestens einen erfassten Bild, der Brechungscharakteristik (D) der Augen (OD, OG) der Person unter den vorgegebenen visuellen Bedingungen.

3. Verfahren nach einem der Ansprüche 1 und 2, ferner aufweisend den folgenden Schritt:
e) Bestimmen, ausgehend von dem mindestens einen erfassten Bild, einer Breite oder einer Leuchtdichteverteilung des Bilds der Netzhautreflexion (IROD1, IROG1, IROD2, IROG2) der Lichtquelle (20) und Bestimmen der Brechungscharakteristik (D) der Augen (OD, OG) der Person in Abhängigkeit von dieser Breite oder dieser Leuchtdichteverteilung.

4. Verfahren nach Anspruch 3, wobei die beiden Augen (OD, OG) gleichzeitig beleuchtet werden, wobei die Bilder der Netzhautreflexionen der Lichtquelle (20) auf der Netzhaut der beiden Augen (OD, OG) gleichzeitig erfasst werden, und bei Schritt e) die Breite oder die Leuchtdichteverteilung des Bilds der Netzhautreflexion (IROD1, IROG1, IROD2, IROG2) der Lichtquelle (20) zumindest ausgehend von den Signalen bezogen auf die Netzhautreflexionen der beiden Augen (OD, OG) bestimmt wird, die gleichzeitig in der Haltung des Kopfes der Person bezogen auf die Lichtquelle (20) erfasst werden.

5. Verfahren nach Anspruch 4, wobei ein Wert der Ametropie der Augen (OD, OG) der Person von der Breite (L) oder der Leuchtdichteverteilung des Bilds der Netzhautreflexion (IROD1, IROG1, IROD2, IROG2) der Lichtquelle (20) abgeleitet wird.

6. Verfahren nach einem der Ansprüche 1 und 2, wobei, wobei mindestens zwei Bilder der Netzhautreflexion (IROD1, IROG1, IROD2, IROG2) der Lichtquelle (20) auf der Netzhaut der beiden Augen (OD, OG) der Person erfasst werden, das Verfahren ferner den folgenden Schritt aufweist:
e') Bestimmen, ausgehend von den mindestens zwei erfassten Bildern, einer Bewegung des Bilds der Netzhautreflexion (IROD1, IROG1, IROD2, IROG2) der Lichtquelle (20),
wobei die Brechungscharakteristik (D) der Augen (OD, OG) der Person in Abhängigkeit von dieser Bewegung bestimmt wird.

7. Verfahren nach Anspruch 6, wobei
die relativen Haltungen des Kopfes der Person bezogen auf die Lichtquelle (20) bei den zwei Bilderfassungen verschieden sind,
bei Schritt c) bei den Bilderfassungen mindestens ein Haltungsparameter (DD, DG, AHD, AHG, AVD, AVG) des Kopfes der Person in dem Bezugssystem (O, X, Y, Z) der Bilderfassungsvorrichtung (10) gemessen wird,
bei Schritt e') die bestimmte Bewegung des Bilds der Netzhautreflexion mit der Differenz der relativen Haltungen des Kopfes der Person bezogen auf die Lichtquelle (20) zwischen den zwei Bilderfassungen in Abhängigkeit von dem Haltungsparameter (DD, DG, AHD, AHG, AVD, AVG) des Kopfes verglichen wird, der bei Schritt c) gemessen wird,
wobei die Brechungscharakteristik (D) der Augen (OD, OG) der Person von diesem Vergleich abgeleitet wird.

8. Verfahren nach Anspruch 7, wobei das Anzeichen für die Ametropie der Augen (OD, OG) der Person von dem Vergleich abgeleitet wird.

9. Verfahren nach einem der Ansprüche 6 bis 8, wobei die beiden Augen (OD, OG) gleichzeitig beleuchtet werden, wobei die Bilder der Netzhautreflexionen der Lichtquelle (20) auf der Netzhaut der beiden Augen (OD, OG) gleichzeitig erfasst werden, und wobei bei Schritt e') die Bewegung des Bilds der Netzhautreflexion (IROD1, IROG1, IROD2, IROG2) der Lichtquelle (20) zumindest ausgehend von den Signalen bezogen auf die Netzhautreflexionen der beiden Augen (OD, OG) bestimmt wird, die gleichzeitig in jeder Haltung des Kopfes der Person bezogen auf die Lichtquelle (20) erfasst werden.

10. Verfahren nach einem der Ansprüche 1 bis 9, wobei bei der Bilderfassung ein Träger (30), auf dem die Lichtquelle (20) und die Bilderfassungsvorrichtung (10) angebracht sind, von der Person in der Hand getragen wird.

11. Verfahren nach einem der Ansprüche 1 bis 10, aufweisend einen Schritt des Überprüfens, vor oder nach dem Bilderfassungsschritt, dass das mindestens eine Bild erfasst wird, wenn die Person in die Nähe, innerhalb eines bestimmten Bereichs, einer Pupille (11) der Bilderfassungsvorrichtung (10) schaut.

12. Verfahren nach einem der Ansprüche 3 bis 11, wobei bei einem Schritt, welcher der Erfassung mindestens eines Bilds vorangeht, eine Sehkorrekturausrüstung auf das Gesicht der Person gesetzt wird und wobei die Signale, die für das erfasste Bild repräsentativ sind, und/oder die Brechungscharakteristik, die von diesen Signalen abgeleitet wird, in Abhängigkeit von der Stärke und der Abweichung der Sehkorrekturausrüstung korrigiert werden.

13. Verfahren nach einem der Ansprüche 1 bis 12, wobei bei Schritt c) die Richtung (DVD, DVG) des Blicks der Person ausgehend von den Signalen berechnet wird, die für die Bilder der Hornhautreflexionen der beiden Augen (OD, OG) repräsentativ sind.

14. Verfahren nach einem der vorhergehenden Ansprüche, aufweisend die folgenden Schritte:

    r) vor oder während der Erfassung mindestens eines Bilds, Anzeigen eines Bezugsbilds vor den Augen der Person, das eine Unschärfe aufweist, die einer Tiefpassfilterung durch einen Filter einer bestimmten Grenzfrequenz der Raumfrequenzen dieses Bezugsbilds entspricht,
    t) Speichern der bestimmten Brechungscharakteristik in Zusammenhang mit dem Grenzfrequenzwert des Tiefpassfilters, der bei Schritt r) genutzt wird, um das Bezugsbild zu filtern,
    u) Wiederholen zumindest der Schritte b), c), d), f), r) und t) oder der Schritte b), c), i), j), r) und t) für verschiedene Grenzfrequenzen des Filters des Bezugsbilds.

15. Verfahren nach einem der Ansprüche 1 und 2, wobei

    - beim Bilderfassungsschritt nacheinander mindestens zwei Bilder erfasst werden, die jeweils die Bilder der zwei Netzhautreflexionen der Lichtquelle auf den Netzhäuten der beiden Augen der Person aufweisen,
    - ausgehend von einem dieser beiden Bilder die Brechungscharakteristik eines der beiden Augen der Person und ausgehend von dem anderen Bild die Brechungscharakteristik des anderen Auges der Person bestimmt wird.

16. Vorrichtung zur automatischen Messung mindestens einer Brechungscharakteristik der beiden Augen (OD, OG) einer Person, aufweisend:

    - einen Träger (30),
    - mindestens eine Lichtquelle (20), die auf diesem Träger (30) angebracht ist, um mindestens eins der Augen (OD, OG) der Person zu beleuchten,
    - mindestens eine Bilderfassungsvorrichtung (10), die auf diesem Träger (30) angebracht ist, die geeignet ist, mindestens ein Bild der Netzhautreflexion (IROD1, IROG1, IROD2, IROG2) der Lichtquelle (20) auf der Netzhaut jedes Auges der Person zu erfassen und jedes erfasste Bild in ein Signal umzuwandeln, das dafür repräsentativ ist, wobei die Position der Lichtquelle (20) in einem Bezugssystem (O, X, Y, Z), das mit der Bilderfassungsvorrichtung (10) verbunden ist, bekannt ist,
    - Mittel zum Messen mindestens eines Blickrichtungsparameters, der mit der Blickrichtung der Person in einem Bezugssystem verbunden ist, das mit deren Kopf verbunden ist, der ausschließlich aus einem Haltungsparameter (DD, DG, H, AHD, AHG, AVD, AVG) des Kopfes der Person besteht, der gemessen wird, wenn die Person eine Pupille der Bilderfassungsvorrichtung mit dem Blick fixiert, der für das Absenken des Blicks zu der Pupille der Bilderfassungsvorrichtung repräsentativ ist, wobei dieser Blickrichtungsparameter visuellen Bedingungen entspricht und der Haltungsparameter des Kopfes (DD, DG, H, AHD, AHG, AVD, AVG) mindestens einen der folgenden Parameter umfasst:

- einen Abstand (DD, DG) zwischen dem Kopf oder einem der Augen (OD, OG) der Person und der Bilderfassungseinrichtung,
- einen vertikalen Abstand (H) zwischen der Pupille der Bilderfassungsvorrichtung (10) und einer geneigten anatomische Ebene (PFI), wenn sich die Person in einer natürlichen Haltung befindet und ihren Blick in Richtung der Bilderfassungsvorrichtung lenkt, die einer anatomischen Horizontebene (PFH) entspricht, die im Wesentlichen horizontal verläuft, wenn sich die Person in einer natürlichen Haltung befindet und zum Horizont schaut,
- einen Abstand zwischen einer Pupille der Bilderfassungsvorrichtung (10) und der anatomischen Horizontebene (PFH), die im Wesentlichen horizontal verläuft, wenn sich die Person in einer natürlichen Haltung befindet und zum Horizont schaut,
- einen ersten Neigungswinkel (AVD, AVG) der Bilderfassungsebene (PCI) bezogen auf eine erste Bezugsebene (PREFD1, PREFG1), die senkrecht zu der Geraden verläuft, die eins der Augen (OD, OG) der Person und die Pupille der Bilderfassungsvorrichtung (10) verbindet, um eine horizontale Achse (X') dieser ersten Bezugsebene, und
- einen zweiten Neigungswinkel (AHD, AHG) der Bilderfassungsebene (PCI) bezogen auf diese erste Bezugsebene (PREFD1, PREFG1) um eine Achse dieser ersten Bezugsebene (PREFD1, PREFG1), die senkrecht zu der horizontalen Achse (X') verläuft.
- eine Recheneinrichtung, die geeignet ist, ausgehend von dem Signal, das für das mindestens eine erfasste Bild repräsentativ ist, die Brechungscharakteristik (D) der Augen (OD, OG) der Person zu bestimmen und den mindestens einen Blickrichtungsparameter, der mit der Blickrichtung der Person verbunden ist, in Zusammenhang mit der Brechungscharakteristik zu speichern, die ausgehend von den Bildern bestimmt wird, die unter den visuellen Bedingungen erfasst werden, die dieser Blickrichtung entsprechen.

**Claims**

1. A method of automatically measuring at least one refractive characteristic of both eyes (OD, OG) of an individual, the method comprising the following steps:

a) adjusting at least one posture of the individual's head in accordance with the wishes of said individual, in a frame of reference (O, X, Y, Z) associated with an image capture device (10) that is adapted to convert each captured image into a signal that is representative thereof;
b) illuminating both eyes (OD, OG) by means of at least one light source (20) of known position in said frame of reference (O, X, Y, Z) associated with the image capture device (10);
c) measuring at least one gaze direction parameter associated with the gaze direction of the individual in a frame of reference associated with the individual's head this gaze direction parameter consisting exclusively in a posture parameter (DD, DG, H, AHD, AHG, AVD, AVG) of the individual's head, the posture parameter being measured while the individual is gazing at the pupil of the image capture device and representative of the gaze being lowered towards the pupil of the image capture device, this head posture parameter (DD, DG, H, AHD, AHG, AVD, AVG) comprising at least one of the following parameters

- a distance (DD, DG) between the head or one of the individual's eyes (OD, OG) and the image capture device,.
- • a vertical distance (H) between a pupil of the image capture device (10) and an anatomical plane (PFI) of the individual that is inclined when the individual is in a natural posture and is looking towards the image capture device, said plane corresponding to a horizon anatomical plane (PFH) that is substantially horizontal when the individual is in a natural posture and looking at the horizon;

• a distance between a pupil of the image capture device (10) and the horizon anatomical plane (PFH) that is substantially horizontal when the individual is in a natural posture and looking at the horizon;
• a first angle of inclination (AVD, AVG) of the image capture plane (PCI) relative to a first reference plane (PREFD1, PREFG1) perpendicular to the line connecting one of the individual's eyes (OD, OG) with the pupil of the image capture device (10), about a horizontal axis (X') of said first reference plan, and

• a second angle of inclination (AHD, AHG) of the image capture plane (PCI) relative to said first reference plane (PREFD1, PREFG1) about an axis of said first reference plane (PREFD1, PREFG1) perpendicular to said horizontal axis (X');

d) using said image capture device (10) to capture at least one image (IROD1, IROG1, IROD2, IROG2) of the retinal reflection of said light source (20) on the retinas of both eyes (OD, OG) of the individual, corresponding to the gaze direction parameter measured in step c);

f) from said at least one image captured in step d), determining said refractive characteristic (D) for the eyes (OD, OG) of the individual; and

h) storing the gaze direction parameter associated with the gaze direction of the individual as measured during the image capture of step d) in association with the determined refractive characteristic deduced from the images captured under the vision conditions corresponding to said gaze direction.

2. A method of automatically measuring at least one refractive characteristic of both eyes (OD, OG) of an individual under predefined vision conditions, the method comprising the following steps:

a) adjusting at least one posture of the individual's head in accordance with the wishes of said individual, in a frame of reference (O, X, Y, Z) associated with an image capture device (10) adapted to convert each captured image into a signal that is representative thereof;

b) illuminating both eyes (OD, OG) by means of at least one light source (20) of known position in said frame of reference (O, X, Y, Z) associated with the image capture device (10);

c) measuring at least one gaze direction parameter associated with the gaze direction of the individual in a frame of reference associated with the individual's head, this gaze direction parameter consisting exclusively in a posture parameter (DD, DG, H, AHD, AHG, AVD, AVG) of the individual's head, the posture parameter being measured while the individual is gazing at the pupil of the image capture device and representative of the gaze being lowered towards the pupil of the image capture device, this head posture parameter (DD, DG, H, AHD, AHG, AVD, AVG) comprising at least one of the following parameters

- a distance (DD, DG) between the head or one of the individual's eyes (OD, OG) and the image capture device,.

- • a vertical distance (H) between a pupil of the image capture device (10) and an anatomical plane (PFI) of the individual that is inclined when the individual is in a natural posture and is looking towards the image capture device, said plane corresponding to a horizon anatomical plane (PFH) that is substantially horizontal when the individual is in a natural posture and looking at the horizon;

• a distance between a pupil of the image capture device (10) and the horizon anatomical plane (PFH) that is substantially horizontal when the individual is in a natural posture and looking at the horizon;

• a first angle of inclination (AVD, AVG) of the image capture plane (PCI) relative to a first reference plane (PREFD1, PREFG1) perpendicular to the line connecting one of the individual's eyes (OD, OG) with the pupil of the image capture device (10), about a horizontal axis (X') of said first reference plan, and

• a second angle of inclination (AHD, AHG) of the image capture plane (PCI) relative to said first reference plane (PREFD1, PREFG1) about an axis of said first reference plane (PREFD1, PREFG1) perpendicular to said horizontal axis (X');

i) comparing the gaze direction parameter measured in step c) with a range of values corresponding to the predefined vision conditions and, if the value of said gaze direction parameter (H) as measured in step c) is situated in said range corresponding to said predefined vision conditions, triggering the capture by said image capture device (10) of at least one image (IROD1, IROG1, IROD2, IROG2) of the retinal reflection of said light source (20) on the retinas of both eyes (OD, OG) of the individual, the image being captured in an image capture plane (PCI); and

j) determining from said at least one captured image, said refractive characteristic (D) of the eyes (OD, OG) of the individual in said predefined vision conditions.

3. A method according to claim 1 or claim 2, further including the following step:

e) determining from said at least one captured image a width or a luminance distribution of the image (IROD1, IROG1, IROD2, IROG2) of the retinal reflection of the light source (20), and determining said refractive characteristic (D) of the individual's eyes (OD, OG) as a function of said width or said luminance distribution.

4. A method according to claim 3, wherein both eyes (OD, OG) are illuminated simultaneously, and the images of the retinal reflections of the light source (20) on the retinas of both eyes (OD, OG) are captured simultaneously, and in step e), the width or the luminance distribution of the image (IROD1, IROG1, IROD2, IROG2) of the retinal reflection of the light source (20) is determined at least from said signals relating to the retinal reflections of both eyes (OD, OG) as captured simultaneously in said posture of the individual's head relative to said light source (20).

5. A method according to claim 4, wherein a value is deduced for the ametropia of each of the individual's eyes (OD, OG) from the width (L) or from the luminance distribution of the image (IROD1, IROG1, IROD2, IROG2) of the retinal reflection of the light source (20).

6. A method according to claim 1 or claim 2, wherein at least two images (IROD1, IROG1, IROD2, IROG2) are captured of the retinal reflections of said light source (20) on the retina of each of the individual's two eyes (OD, OG), and the method further includes the following step:
   e') determining from said at least two captured images a movement of the image (IROD1, IROG1, IROD2, IROG2) of the retinal reflection of the light source (20) ;
   said refractive characteristic (D) of each of the individual's eyes (OD, OG) being determined as a function of said movement.

7. A method according to claim 6, wherein:

   for the posture of the individual's head relative to said light source (20) differing during the two image captures;
   in step c), during said image captures, at least one posture parameter (DD, DG, AHD, AHG, AVD, AVG) of the individual's head is measured in the frame of reference (O, X, Y, Z) of the image capture device (10); and
   in step e'), the determined movement of the image of the retinal reflection is compared with the difference in posture of the individual's head relative to the light source (20) between the two image captures as a function of said head posture parameter (DD, DG, AHD, AHG, AVD, AVG) measured in step c);

said refractive characteristic (D) of the individual's eyes (OD, OG) being deduced from the comparison.

8. A method according to claim 7, wherein the sign of the ametropia of each of the individual's eyes (OD, OG) is deduced from said comparison.

9. A method according to any one of claims 6 to 8, wherein both eyes (OD, OG) are illuminated simultaneously, and the images of the retinal reflections of the light source (20) on the retinas of both eyes (OD, OG) are captured simultaneously, and wherein, in step e'), the movements of the images (IROD1, IROG1, IROD2, IROG2) of the retinal reflections of the light source (20) are determined at least from said signals relating to the retinal reflections of both eyes (OD, OG) as captured simultaneously in each posture of the individual's head relative to said light source (20).

10. A method according to any one of claims 1 to 9, wherein, during image capture, a support (30) on which said light source (20) and said image capture device (10) are mounted is carried by said individual in the hand.

11. A method according to any one of claims 1 to 10, including a verification step before or after the image capture step for verifying that said at least one image is captured while the individual is looking in the vicinity of a pupil (11) of the image capture device (10), i.e. looking within a determined area in the vicinity of the pupil.

12. A method according to any one of claims 3 to 11, wherein, during a preliminary step before capturing at least one image, vision correction equipment is placed on the face of said individual and wherein the signals representing said captured image and/or the refractive characteristic deduced from said signals is corrected as a function of the power and the deflection of the vision correction equipment.

13. A method according to any one of claims 1 to 12, wherein, in step c), the individual's gaze direction (DVD, DVG) is calculated from the signals representative of the images of the corneal reflections of both eyes (OD, OG).

14. A method according to any one of the preceding claims, comprising the following steps:

   r) before or during the capture of at least one image, displaying a reference image before the individual's eyes, the reference image presenting fuzziness corresponding to lowpass filtering by a frequency filter of given cutoff for filtering the spatial frequencies of said reference image;
t) storing in memory the refractive characteristic as determined in association with the value of the cutoff frequency of the lowpass filter used in step r) for filtering the reference image; and
u) reiterating at least steps b), c), d), f), r), and t) or steps b), c), i), j), r) and t)for different cutoff frequencies of the reference image filter.

15. A method according to any one of claims 1 and 2, wherein:

> • during said image capture step, at least two images are captured in succession, each corresponding to the images of the two retinal reflections of said light source on the retinas of both eyes of the individual; and
> • from one of said two images, determining said refractive characteristic of one of the two eyes of the individual, and from the other image, determining the refractive characteristic of the other eye of the individual.

16. A device for automatically measuring at least one refractive characteristic of both eyes (OD, OG) of an individual, the device comprising:

> • a support (30);
> • at least one light source (20) mounted on the support (30) in such a manner as to illuminate at least one of the individual's eyes (OD, OG);
> • at least one image capture device (10) mounted on said support (30), adapted to capture at least one image (IROD1, IROG1, IROD2, IROG2) of the retinal reflection of said light source (20) on the retina of each eye of the individual and to convert each captured image into a signal that is representative thereof, said light source (20) being of known position in a frame of reference (O, X, Y, Z) associated with said image capture device (10);
> • means for measuring at least one gaze direction parameter associated with the individual's gaze direction in a frame of reference associated with the individual's head, this gaze direction parameter consisting exclusively in a posture parameter (DD,DG, H, AHD, AHG, AVD, AVG) of the individual's head, the posture parameter being measured while the individual is gazing at the pupil of the image capture device and representative of the gaze being lowered towards the pupil of the image capture device, the gaze direction parameter corresponding to vision conditions and the head posture parameter (DD, DG, H, AHD, AHG, AVD, AVG) comprising at least one of the following parameters :
>
>> - a distance (DD, DG) between the head or one of the individual's eyes (OD, OG) and the image capture device,.
- • a vertical distance (H) between a pupil of the image capture device (10) and an anatomical plane (PFI) of the individual that is inclined when the individual is in a natural posture and is looking towards the image capture device, said plane corresponding to a horizon anatomical plane (PFH) that is substantially horizontal when the individual is in a natural posture and looking at the horizon;

• a distance between a pupil of the image capture device (10) and the horizon anatomical plane (PFH) that is substantially horizontal when the individual is in a natural posture and looking at the horizon;
• a first angle of inclination (AVD, AVG) of the image capture plane (PCI) relative to a first reference plane (PREFD1, PREFG1) perpendicular to the line connecting one of the individual's eyes (OD, OG) with the pupil of the image capture device (10), about a horizontal axis (X') of said first reference plan, and
• a second angle of inclination (AHD, AHG) of the image capture plane (PCI) relative to said first reference plane (PREFD1, PREFG1) about an axis of said first reference plane (PREFD1, PREFG1) perpendicular to said horizontal axis (X').

Fig.1

Fig.2

Fig.3

Fig.4

Fig.5

IOD2   I2   IPG2   IOG2

IPD2

ILD2

IROD2   IROG2

ILG2

Fig.6

OD

PFH

PFI

PCI

30

PREFD1

H   11   20

Z

100

DOD

10

Y

AVD;AVG

DD

X

O   X'

I

Fig.8

——— D=+/- 0,05

------- D=+/- 0,25

— — D=+/- 0,5

—·— D=+/- 1

u

Fig.7

PCI

PREFD1

AHD

100

30

DG

OG

DVG

DOG

AG

Y

11

X

DVD

Z

OD

AD

DOD

10

DD

PREFG1

AHG

y

PREFG1

26

EP 2 498 671 B1

## RÉFÉRENCES CITÉES DANS LA DESCRIPTION

**Documents brevets cités dans la description**

- US 2009079937 A
- US 6616277 B1
- WO 2009024681 A
- WO 2008132356 A
- US 20030169907 A

**Littérature non-brevet citée dans la description**

- **M. KLEIN.** Principles of retinoscopy. *The British Journal of Ophtalmology,* 1944
- Real-time modeling of face déformation for 3D head pose estimation. **M. OKA et al.** Analysis and Modelling of Faces and Gestures. 2005, 308-320
- *Optometry and vision science,* 1997, vol. 174, 472-482